# EUROPEAN PATENT SPECIFICATION

(11) **EP 2 346 389 B1**
(45) Date of publication and mention of the grant of the patent: **22.05.2013**
(21) Application number: 08794792.5
(22) Date of filing: 28.07.2008
(51) Int. Cl.: A61B 5/00, A61B 5/0215, A61B 5/026

(54) **IMPLANTABLE OPTICAL HEMODYNAMIC SENSOR INCLUDING LIGHT TRANSMISSION MEMBER**
IMPLANTIERBARER OPTISCHER HÄMODYNAMISCHER SENSOR MIT LICHTÜBERTRAGUNGSELEMENT
CAPTEUR HÉMODYNAMIQUE OPTIQUE IMPLANTABLE COMPRENANT UN ÉLÉMENT DE TRANSMISSION DE LUMIÈRE

(43) Date of publication of application: 27.07.2011
(73) Proprietor: Medtronic, Inc., Minneapolis, MN 55342-5604 (US)
(72) Inventor: CINBIS, Can, Shoreview MN 55126 (US); O'BRIEN, Richard, James, Hugo MN 55038 (US); CARNEY, James, Kevin, Brooklyn Park MN 55443 (US); DONOFRIO, William, Andover, Minnesota 55304 (US)
(74) Representative: Hughes, Andrea Michelle
(86) International application number: PCT/US2008/009099
(87) International publication number: WO 2010/014053

(56) References cited:
- WO-A-96/25978
- WO-A-2007/100283
- WO-A-2007/110867
- WO-A-2008/017967
- US-A- 5 553 616
- US-A- 5 601 611
- US-A1- 2007 027 371

## Description

### TECHNICAL FIELD

The disclosure relates to medical devices, and, more particularly, to medical devices that monitor one or more physiological parameters of a patient.

### BACKGROUND

Some medical devices may monitor one or more hemodynamic characteristics of a patient, such as the oxygen saturation level of blood of the patient (e.g., arterial blood), the volume of blood supplying a particular tissue site, and the like. Example medical devices that monitor hemodynamic characteristics of a patient include pulse oximeters, blood flow sensors, hematocrit sensors, and tissue perfusion sensors. One type of pulse oximeter, which may also be referred to as an optical perfusion sensor, includes at least one light source that emits light through a portion of blood-perfused tissue of a patient, and an optical detector ("detector") that senses the emitted light that passed through the blood-perfused tissue. An intensity of the light sensed by the detector may be indicative of hemodynamic function of the patient, such as oxygen saturation of blood of the patient.

In some types of pulse oximeters, the one or more light sources may be positioned on the same side of the blood perfused tissue as the detector, such that the detector detects light emitted by the light sources and reflected by blood. This type of pulse oximeter may be referred to as a reflectance-type pulse oximeter. In other types of pulse oximeters, referred to as transmissive-type pulse oximeters, the one or more light sources may oppose the detector, such that the detector senses light that is transmitted through the blood perfused tissue.
US 5,553,616, US 5,601,611, US 2007/027371, US 6,048,359, WO 96/25978, WO 2007/110867 and WO 2007/100283 all teach devices that monitor physiological parameters of a patient using light transmission and detection.

### SUMMARY

The invention provides an implantable medical system as defined by claim 1. In general, the disclosure is directed to an implantable medical device (IMD) that includes an optical hemodynamic sensor, such as a pulse oximeter or a tissue perfusion sensor. The hemodynamic sensor may include at least one optical emitter (or light source) and at least one detector. In some examples, the optical emitter is optically coupled to at least one light transmission member that extends from a biocompatible, implantable housing of the IMD. The light transmission member may guide light emitted by the optical emitter to blood-perfused tissue of patient. In some examples, the detector may be optically coupled to at least one light transmission member that extends from the housing of the IMD. The light transmission member may help collect light emitted by the optical emitter and transmit the collected light to the detector. In some examples, the light transmission members may be substantially flexible, such that the distal ends of the light transmission members are movable with respect to the housing of the IMD. In other examples, the light transmission members may be substantially rigid, such that the distal ends of the light transmission members are substantially fixed relative to the housing of the IMD.

In other examples, at least one optical emitter of the hemodynamic sensor is carried by an extension member that extends from a housing of the IMD. In addition, in some examples, at least one detector of the hemodynamic sensor is carried by an extension member that extends from the housing of the IMD. The extension member may comprise one or more electrically conductive members that electrically couple the optical emitter or the detector to one or more components within the IMD housing. For example, the extension member may comprise a flexible circuit that extends from the housing of the IMD and is electrically coupled to a controller or other components within the IMD housing.

A light transmission member that extends from the housing of the IMD and is optically coupled to an optical emitter or a detector of a hemodynamic sensor may help define a hemodynamic sensor that is adaptable to different implantation sites within the patient. In some examples, a clinician may customize the configuration of the hemodynamic sensor to a particular implant site within the patient by manipulating the position of the one or more light transmission members that are optically coupled to the optical emitter and/or detector relative to a housing of the IMD. The light transmission members have a smaller cross-sectional size than the housing of the IMD, which may permit the light transmission members to be implanted proximate to vasculature or another blood mass of the patient, even if the housing of the IMD is too large or otherwise unsuitable for implantation proximate to the blood mass. This may facilitate implantation of the IMD such that the optical emitter and detector of the hemodynamic sensor may emit and sense light proximate to a blood mass of the patient.

In one aspect, the disclosure is directed to an implantable medical system comprising an implantable housing, a light transmission member extending from the housing, an optical emitter coupled to the housing, and a detector coupled to the housing. The detector generates a signal indicative of an intensity of light emitted by the optical emitter and transmitted through the patient's blood to the detector. At least one of the optical emitter or the detector are optically coupled to the light transmission member.

In another aspect, the disclosure is directed to an implantable medical system comprising an implantable housing, a light transmission member extending from the housing, means for emitting light, wherein the means for emitting light is coupled to the housing, and means for sensing light, wherein the means for sensing light is coupled to the housing. The means for sensing light is configured to generate a signal indicative of an intensity of light emitted by the means for emitting light and transmitted to the means for sensing light. At least one of the means for emitting light or the means for sensing light are optically coupled to the light transmission member.

In another aspect, the disclosure is directed to an implantable medical system comprising an implantable housing, an optical emitter that emits light, a detector coupled to the housing, and an optically conductive member optically coupled to the optical emitter, where the light emitted by the optical emitter propagates through optically conductive member in a direction away from the housing. The detector generates a signal indicative of an intensity of the light that has passed through the optically conductive member and is transmitted to the detector.

In another aspect, the disclosure is directed to a method comprising transmitting light from an implantable optical emitter coupled to an implantable housing, and receiving the light at an implantable detector coupled to the housing, where at least one of the optical emitter or the detector are optically coupled to a light transmission member that extends from the housing. In some examples, transmitting the light from the implantable optical emitter comprises transmitting light from the housing to a distal end of the light transmission member. In some examples, receiving the light at the detector comprises receiving the light at a distal end of the light transmission member and guiding the light to the detector via the light transmission member.

In another aspect, the disclosure is directed to an implantable medical system comprising means for transmitting light from an implantable optical emitter coupled to an implantable housing, and means for receiving the light at an implantable detector coupled to the housing, where at least one of the optical emitter or the detector are optically coupled to a light transmission member that extends from the housing.

In another aspect, the disclosure is directed to an implantable medical system comprising an implantable housing, an extension member extending from the housing, an optical emitter that emits light, and a detector that generates a signal indicative of an intensity of light emitted by the optical emitter and transmitted to the detector, the signal indicating a hemodynamic characteristic of a patient. At least one of the optical emitter or the detector are carried by the extension member outside of the housing.

In another aspect, the disclosure is directed to an implantable medical system comprising an implantable housing, an optical emitter that emits light, a detector that generates a signal indicative of an intensity of light emitted by the optical emitter and transmitted to the detector, a flexible circuit electrically coupled to at least one of the optical emitter or the detector, wherein the flexible circuit extends away from the housing, and a processor disposed within the implantable housing. The processor receives the signal generated by the detector and determines a hemodynamic characteristic of a patient based on the signal. The flexible circuit electrically couples the at least one of the optical emitter or the detector to the processor.

In another aspect, the disclosure is directed to a method comprising emitting light from an implantable optical emitter, receiving the light at an implantable detector that generates a signal indicative of an intensity of light emitted by the optical emitter and transmitted to the detector, wherein at least one of the optical emitter or the detector are carried by an extension member that extends from a housing of an implantable medical device, the at least one of the optical emitter or detector being located outside of the housing, and receiving the signal at a processor disposed within the housing of the implantable medical device. The processor determines a hemodynamic characteristic of a patient based on the signal.

In another embodiment, the invention is directed to a computer-readable medium containing instructions. The instructions cause a programmable processor to perform any one or more of the techniques described herein.

The details of one or more aspects of the disclosure are set forth in the accompanying drawings and the description below, but the scope of the invention is defined by the claims.

### BRIEF DESCRIPTION OF DRAWINGS

Some of the drawings are shown for background teaching only. The invention is as defined by the claims.

FIG. 1 is a conceptual diagram illustrating an example system that includes an implantable medical device (IMD) comprising an implantable hemodynamic sensor.

FIG. 2 is a conceptual diagram illustrating an example therapy system that includes an IMD that delivers therapy to a patient, where the IMD includes a hemodynamic sensor.

FIG 3 is a functional block diagram of an example IMD that includes a hemodynamic sensor.

FIG. 4 is a functional block diagram of an example medical device programmer.

FIGS. 5 and 6 are conceptual illustrations of example IMDs that each include a light transmission member coupled to one or more optical emitters of a hemodynamic sensor.

FIG. 7 is a conceptual illustration of an example IMD that includes a light transmission member with a light deflection member that helps direct light emitted by an optical emitter in a particular direction.

FIG. 8 is a conceptual illustration of an example IMD that includes a hemodynamic sensor.

FIG. 9 is a conceptual illustration of an example IMD that includes a hemodynamic sensor optically coupled to a plurality of light transmission members.

FIGS. 10-12 are conceptual illustrations of examples implantable transmissive-type hemodynamic sensors.

FIG. 13 is a conceptual illustration of an example IMD that includes a hemodynamic sensor, where an optical emitter of the sensor is carried by an extension member that extends from a housing of the IMD.

FIG. 14 is a conceptual illustration of an example IMD that includes a hemodynamic sensor, where an optical emitter and detector of the sensor are carried by respective extension members that extends from a housing of the IMD.

### DETAILED DESCRIPTION

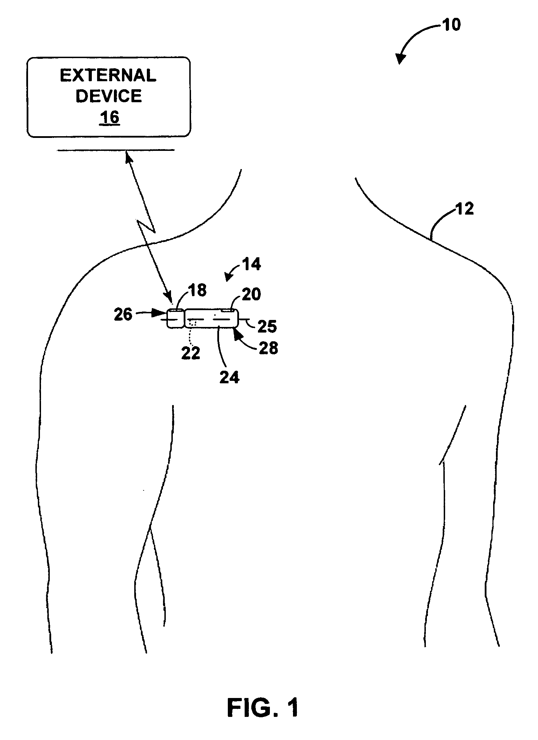

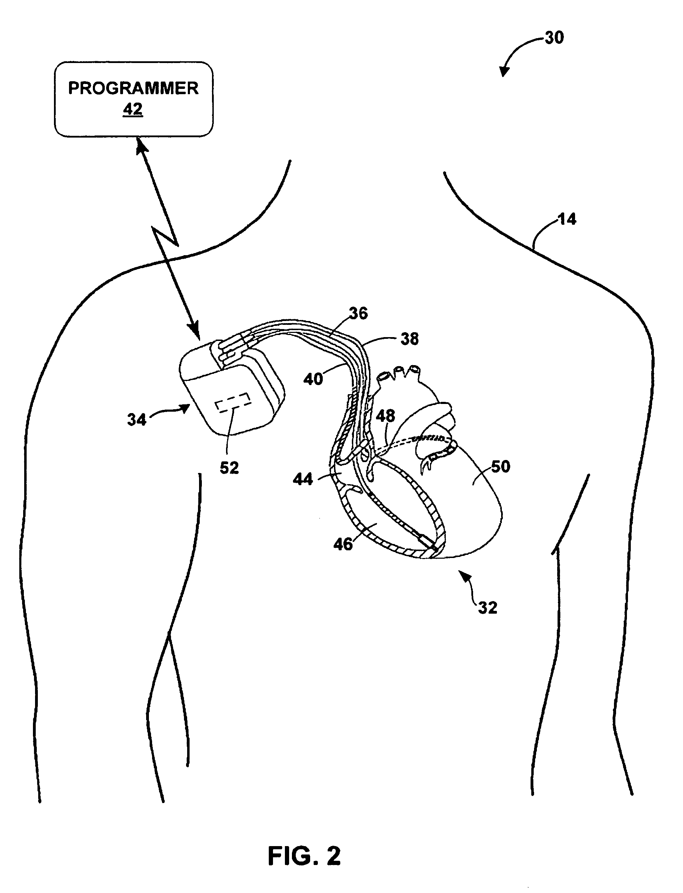

FIG. 1 is a conceptual diagram illustrating an example system 10 that may be used to monitor one or more physiological parameters of patient 12, such as an oxygen saturation level of blood of patient 12 or other hemodynamic characteristics of patient 12. Patient 12 ordinarily, but not necessarily, will be a human. Monitoring system 10 includes implantable medical device (IMD) 14 and external device 16. IMD 14 may be, for example, an implantable cardiac monitor that does not provide therapy (e.g., stimulation therapy) to patient 12. IMD 14 may also be referred to as an implantable monitor. In other examples, e.g., as described with respect to FIG. 2, IMD 14 may be configured to deliver stimulation to the heart of patient 12 or to deliver another type of therapy to patient 12 (e.g., delivery of a therapeutic agent). Neither IMD 14, external device 16 nor any of the figures shown herein are drawn to any particular scale.

In the example shown in FIG. 1, IMD 14 is implanted within a subcutaneous tissue layer of patient 12. Due to its relatively small size, a clinician may implant monitor 14 through a relatively small incision in the patient's skin, or percutaneously, e.g., via an introducer. In other examples, IMD 14 may be implanted within other tissue sites, such as a submuscular location. IMD 14 may be a temporary diagnostic tool employed to monitor one or more physiological parameters of patient 12 for a relatively short period of time (e.g., days or weeks), or may be used on a more permanent basis, such as to control therapy delivery to patient 12. In some examples of the latter use of IMD 14, a separate therapy delivery device, such as a fluid delivery device, pacemaker, cardioverter-defibrillator, or neurostimulator, may be implanted within patient 12. The therapy delivery device may communicate with IMD 14 via a wired connection or via wireless communication techniques. In other examples, as previously described, IMD 14 may be incorporated in a common housing with a therapy delivery device.

IMD 14 includes electrodes 18, 20 that sense electrical activity of patient's heart. For example, IMD 14 may generate an electrogram (EGM) or electrocardiogram (ECG) based on signals from electrodes 18, 20. While other types of electrical signals of the heart of patient 12 are contemplated, EGM signals are primarily referred to throughout the remainder of the disclosure for purposes of illustration. Accordingly, ECG or other types of cardiac signals may be acquired and stored in accordance with the techniques, devices, and systems described herein. Electrodes 18, 20 may be positioned any suitable distance from each other. In the example shown in FIG. 1, electrodes 18, 20 are coupled to an outer housing 24 of IMD 14. In other examples, electrodes 18, 20 may be coupled to leads that extend from outer housing 24 of IMD 14. In some examples, housing 24 may comprise a biocompatible and hermetic housing.

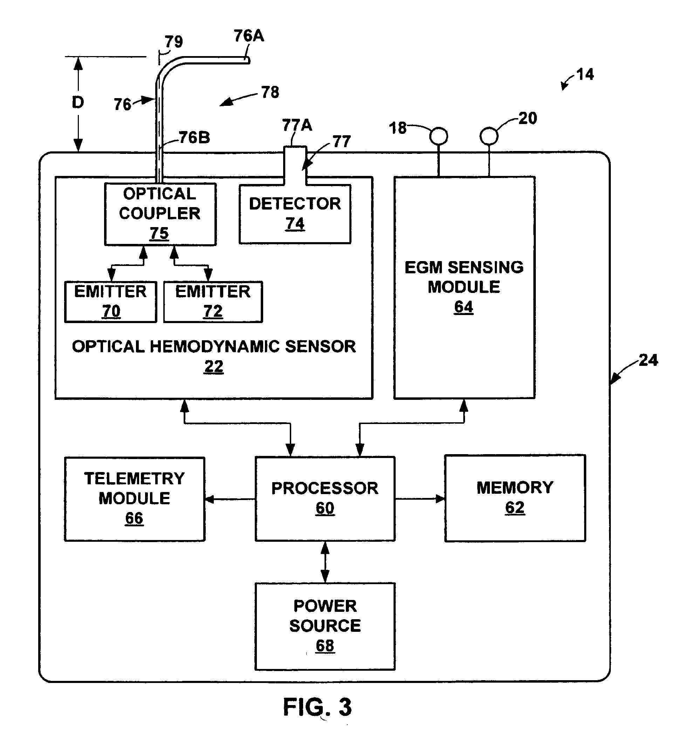

IMD 14 further includes implantable optical hemodynamic sensor 22 that generates an electrical signal indicative of the arterial blood oxygen saturation level in a tissue site proximate to hemodynamic sensor 22. In some examples, hemodynamic sensor 22 may comprise a pulse oximeter, blood flow sensor, hematocrit sensor or a tissue perfusion sensor. In some examples, hemodynamic sensor 22 generates an electrical signal that changes as a function of the blood oxygen saturation level of blood of patient 12, such that the signal from hemodynamic sensor 22 may be used to determine relative changes in the patient's blood oxygen saturation level. The blood oxygen saturation level may be indicative of various hemodynamic characteristics, such as pulmonary function or blood pressure of patient 12, which may indicate the health status of patient 12. Examples of implantable hemodynamic sensors that IMD 14 may include are described below with reference to FIGS. 3 and 5-12. Although hemodynamic sensor 22 and electrodes 18, 20 are shown to be on different sides of housing 24 of IMD 14, in other examples, hemodynamic sensor 22 may be on the same side of housing 24 with at least one of the electrodes 18, 20.

In the example shown in FIG. 1, hemodynamic sensor 22 includes at least one detector and at least one optical emitter (or light source). As described in further detail below with reference to FIGS. 3 and 5-9, the detector and/or optical emitter may be optically coupled to a light transmission member that extends from housing 24 of IMD 14. That is, light emitted by the optical emitter may be introduced into the light transmission member and propagate through the light transmission member away from housing 24, which may permit the optical emitter to emit light at a location remote from the optical emitter. The light transmission member may carry light along its length. Thus, the light generated by the optical emitter may traverse through the light transmission member, which may guide the emitted light to a tissue site, e.g., proximate to vasculature or another blood mass of patient 12.

In examples in which the detector is optically coupled to a light transmission member, the detector may receive light at a location remote from the detector via the light transmission member. For example, light incident on a distal end of the light transmission member or another light receiving portion of the light transmission member may propagate through the light transmission member to the detector. In this way, the light transmission member may guide light from tissue proximate to vasculature or another blood mass of patient 12 to the detector, which may be located on the device housing. As described in further detail below, the light transmission members described herein that extend from housing 24 of IMD 14 may be useful for effectively positioning the optical emitter and/or detector of hemodynamic sensor 22 proximate to a blood mass of patient 12 without requiring housing 24 to be implanted proximate to the blood mass.

The one or more optical emitters of hemodynamic sensor 22 may emit light at a particular wavelength, and the one or more detectors may each be configured to sense light that emitted from the optical emitter and transmitted through a medium. The medium may be, for example, blood-perfused tissue of patient 12, such as tissue comprising a blood mass (e.g., blood cells in a blood vessel) of patient 12. In some examples, hemodynamic sensor 22 may include at least two optical emitters that emit light at different wavelengths and, in some cases, at least two detectors that are sensitive to different wavelengths of light. The detectors may be configured to generate an electrical signal indicative of an intensity of light emitted by the one or more optical emitters, transmitted through tissue of patient, and sensed by the detector.

In the example shown in FIG. 1, housing 24 includes header 26 and case 28. Case 28 may be hermetically sealed and may enclose various sensing and control circuitry for sensing one or more physiological parameters of patient 12, and, in some cases, a therapy delivery module for delivering therapy to patient 12 (e.g., electrical stimulation or a therapeutic agent). Header 26 may provide a hermetically sealed passage for connecting electrode 18 and hemodynamic sensor 22 to components within case 28. In the example shown in FIG. 1, one or more detectors of hemodynamic sensor 22 are coupled to case 28 of IMD 14. In other examples, one or more detectors of hemodynamic sensor 22 may be positioned on header 26 of IMD 14 or both header 26 and case 28. In other examples of IMD 14, IMD 14 may not include a separate header 26 and case 28.

IMD 14 may be implanted within patient 12 such that hemodynamic sensor 22 is adjacent to blood-perfused tissue. For example, the blood-perfused tissue may be positioned between the one or more detectors and the one or more optical emitters of hemodynamic sensor 22. Hemodynamic sensor 22 may be positioned proximate to tissue that is near vasculature of patient 12 (e.g., one or more blood vessels), but not within a vein, artery, or heart of patient 12. In other examples, hemodynamic sensor 22 may be positioned within a vein or other vasculature of patient 12.

Oxygenated and deoxygenated hemoglobin within blood may unequally absorb different wavelengths of light. The optical properties of blood-perfused tissue may change depending upon the relative amounts of oxygenated and deoxygenated hemoglobin due, at least in part, to their different optical absorption spectra. That is, the oxygen saturation level of the patient's blood may affect the amount of light that is absorbed by a blood mass and the amount of light that is transmitted through the blood-perfused tissue. Accordingly, an electrical signal generated by hemodynamic sensor 22 that indicates the intensity of one or more wavelengths of light detected by the detector of sensor 22 may change based on the relative amounts of oxygenated and deoxygenated hemoglobin in the tissue. That is, the intensity of light that is emitted by the optical emitter of sensor 22 and transmitted through blood may indicate relative blood oxygen saturation levels of patient 12.

At least some of the light transmitted through the blood may be detected by the detector of hemodynamic sensor 22. The optical emitter of hemodynamic sensor 22 may be coupled to a light transmission member that extends away from housing 24. The light transmission member may permit blood-perfused tissue of patient 12 to be disposed between the location at which light is emitted into blood-perfused tissue of patient 12 (e.g., the distal end of the light transmission member) and the detector of hemodynamic sensor 22.

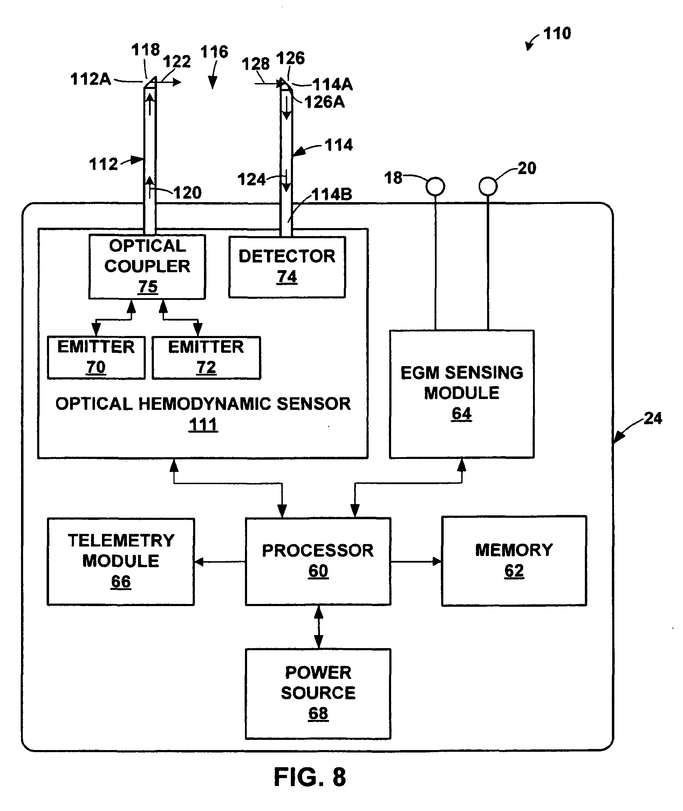

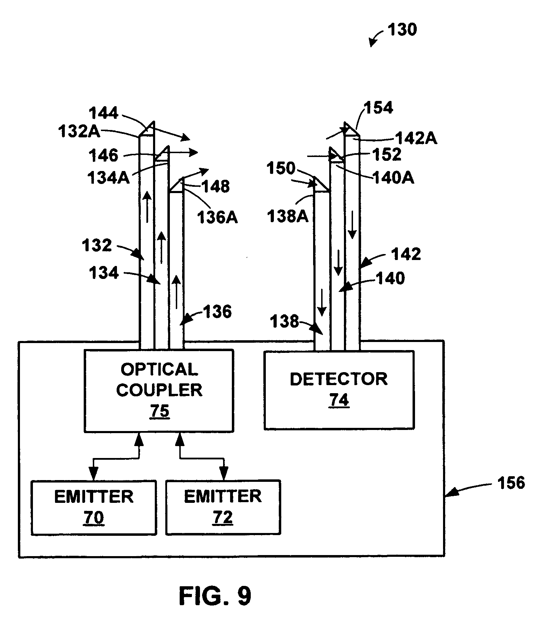

In other examples, as described in further detail below with reference to FIGS. 8 and 9, a detector of hemodynamic sensor 22 may be coupled to one or more light transmission members that extend away from housing 24, and the optical emitter may emit light away from housing 24 toward a distal end of the light transmission member. The detector may be, for example, disposed within housing 24 or otherwise coupled to housing 24. Light emitted by the optical emitter may traverse through blood-perfused tissue of patient 12, and the light transmission member may guide light that is incident on the distal end of the light transmission member to the detector.

In some examples, both the detector and optical emitter of hemodynamic sensor 22 may be coupled to respective light transmission members. As described below with reference to FIG. 8, the light transmission members may be substantially parallel or may have another suitable arrangement that permits light emitted by the optical emitter on or within housing 24 to be guided to a tissue site by a first light transmission member and light emitted by the optical emitter that is transmitted through the tissue to be guided to the detector on or within housing 24 by a second light transmission member.

The arrangement between the detector and optical emitter of hemodynamic sensor 22 described herein defines a transmissive-type hemodynamic sensor because light that is emitted by the optical emitter is transmitted through blood-perfused tissue of patient 12 prior to being received by the detector. In contrast, a hemodynamic sensor including one or more optical emitters positioned on the same side of the blood perfused tissue as the detector, such that the detector detects light emitted by the optical emitters and reflected by blood, may be referred to as a reflectance-type hemodynamic sensor. A detector of an implanted, transmissive-type hemodynamic sensor 22 may sense a greater quantity of light emitted by the optical emitter and transmitted through the patient's blood compared to a reflectance-type hemodynamic sensor. Increasing the overall quantity of light that is emitted by the optical emitter of hemodynamic sensor 22 and sensed by the detector of hemodynamic sensor may help improve the signal to noise ratio of hemodynamic sensor 22.

In some examples, IMD 14 may be implanted within patient 12 such that hemodynamic sensor 22, or at least the detector, faces away from the epidermis of patient 12 in order to help minimize interference from background light, e.g., from outside of the patient's body. In examples in which the detector is coupled to a light transmission member, the portion of the light transmission member that receives light may face away from the epidermis of patient 12. Background light may include light from a source other than the one or more optical emitters of hemodynamic sensor 22. Detection of the background light by the detector of hemodynamic sensor 22 may result in an inaccurate and imprecise reading of the level of blood oxygen saturation of the adjacent tissue.

IMD 14 may be useful for monitoring physiological parameters, such as the EGM and blood oxygen saturation level, of patient 12. Changes in blood oxygenation of the tissue adjacent to hemodynamic sensor 22 may indicate various hemodynamic characteristics of patient 12. For example, hemodynamic characteristics of a cardiac rhythm of patient 12 may be derived from a signal generated by hemodynamic sensor 22. In some cases, the signal generated by hemodynamic sensor 22 may indicate the blood oxygen saturation level of the adjacent tissue or the body of patient 12 as a whole. As used herein, "tissue perfusion" may also refer to the concentration of oxygen in blood within the tissue. Accordingly, tissue perfusion and blood oxygenation levels are interchangeably referred to in the present disclosure.

As described in further detail below with reference to FIG. 3, IMD 14 may include a memory that stores EGM signals and blood oxygen saturation information (e.g., electrical signals generated by hemodynamic sensor 22 or data derived from the electrical signal). In addition or alternatively, IMD 14 may transmit the EGM signals and tissue perfusion information to an external device, such as external device 16. In some examples, IMD 14 may store the blood oxygen saturation information that corresponds in time to the sensed EGM signals (or other cardiac signals), thereby allowing a clinician to determine the patient's cardiac activity at the time a particular blood oxygen saturation level was observed, or to determine the patient's blood oxygen saturation level at the time a particular cardiac activity was observed. Accordingly, the tissue perfusion information and cardiac signal information generated by IMD 14 may be later retrieved and analyzed by a clinician. In some examples, a clinician may retrieve stored EGM and tissue perfusion information from IMD 14 after explanting IMD 14 from patient 12. In other examples, the clinician (or other user) may interrogate IMD 14 with external device 16 via wireless telemetry while IMD 14 remains implanted within patient 12 in order to retrieve stored information from IMD 14.

The physiological parameter values monitored by IMD 14 may provide useful information for diagnosing a patient condition or formulating a treatment plan for patient 12. For example, if patient 12 experiences syncope, e.g., periodic fainting, IMD 14 may be used to determine the physiological parameters that are associated with the syncope. A clinician may review the associated physiological parameters to determine a potential cause of the syncopic events. For example, a clinician may determine whether any patient events occurred based on the recorded signals from hemodynamic sensor 22, and, in some cases, recorded cardiac signals obtained via electrodes 18, 20.

External device 16 may be a handheld computing device or a computer workstation. External device 16 may include a user interface that receives input from a user, such as a clinician. The user interface may include, for example, a keypad and a display, which may for example, be a cathode ray tube (CRT) display, a liquid crystal display (LCD) or LED display. The keypad may take the form of an alphanumeric keypad or a reduced set of keys associated with particular functions. External device 16 can additionally or alternatively include a peripheral pointing device, such as a mouse, via which a user may interact with the user interface. In some embodiments, a display of external device 16 may include a touch screen display, and a user may interact with external device 16 via the display.

A user, such as a physician, technician, or other clinician, may interact with external device 16 to communicate with IMD 14. For example, the user may interact with external device 16 to retrieve physiological or diagnostic information from IMD 14. A user may also interact with external device 16 to program IMD 14, e.g., select values for operational parameters of monitor 14.

For example, the user may use external device 16 to retrieve information from IMD 14 regarding the rhythm of the heart of patient 12 (e.g., determined based on an EGM signal), trends of the heart rhythm over time, or arrhythmia episodes. As another example, the user may use external device 16 to retrieve information from IMD 14 regarding other sensed physiological parameters of patient 12, such as tissue perfusion data, activity, posture, respiration, or thoracic impedance. As another example, the user may use external device 16 to retrieve information from IMD 14 regarding the performance or integrity of IMD 14.

IMD 14 and external device 16 may communicate via wireless communication using any techniques known in the art. Examples of communication techniques may include, for example, low frequency or radiofrequency (RF) telemetry, but other techniques are also contemplated. In some examples, external device 16 may include a programming head that may be placed proximate to the patient's body near the implant site of IMD 14 in order to improve the quality or security of communication between IMD 14 and external device 16.

In other examples, IMD 14 may not include electrodes 18, 20 for monitoring cardiac signals of the heart of patient 12. For example, IMD 14 may only include hemodynamic sensor 22 that monitors hemodynamic function of patient 12, such as the blood oxygen saturation level of arterial blood or blood pressure of patient 12. The light transmission members described herein may be useful with any suitable type of optical sensor that senses light. Accordingly, while IMD 14 including EGM and hemodynamic sensor capabilities is primarily referred to herein, in other examples, the light transmission members coupled to a detector or an optical emitter may be incorporated into other types of optical sensors.

FIG. 2 is a conceptual diagram illustrating an example therapy system 30 that may be used to provide therapy to heart 32 of patient 12. Therapy system 30 includes IMD 34 that provides therapy to patient 12. IMD 34 is coupled to leads 36, 38, and 40, and programmer 42. IMD 34 may be, for example, an implantable pacemaker, cardioverter, and/or defibrillator that provides electrical signals to heart 32 via electrodes coupled to one or more of leads 36, 38, and 40.

Leads 36, 38, 40 extend into the heart 32 of patient 12 to sense electrical activity of heart 32 and/or deliver electrical stimulation to heart 32. In the example shown in FIG. 2, right ventricular (RV) lead 36 extends through one or more veins (not shown), the superior vena cava (not shown), and right atrium 44, and into right ventricle 46. Left ventricular (LV) coronary sinus lead 38 extends through one or more veins, the vena cava, right atrium 44, and into the coronary sinus 48 to a region adjacent to the free wall of left ventricle 50 of heart 32. Right atrial (RA) lead 40 extends through one or more veins and the vena cava, and into the right atrium 44 of heart 32.

IMD 34 may sense electrical signals attendant to the depolarization and repolarization of heart 32 via electrodes (not shown in FIG. 2) coupled to at least one of the leads 36, 38, 40. In some examples, IMD 34 provides pacing pulses to heart 32 based on the electrical signals sensed within heart 32. The configurations of electrodes used by IMD 34 for sensing and pacing may be unipolar or bipolar. IMD 34 may also provide defibrillation therapy and/or cardioversion therapy via electrodes located on at least one of the leads 36, 38, 40. IMD 34 may detect arrhythmia of heart 32, such as fibrillation of ventricles 46, 50, and deliver defibrillation therapy to heart 32 in the form of electrical pulses. In some examples, IMD 34 may be programmed to deliver a progression of therapies, e.g., pulses with increasing energy levels, until a fibrillation of heart 32 is stopped. IMD 34 detects fibrillation employing one or more fibrillation detection techniques known in the art.

IMD 34 includes implantable optical hemodynamic sensor 52, which is similar to implantable hemodynamic sensor 22 described above with respect to FIG. 1. IMD 34 may include features similar to those described with respect to IMD 14. Accordingly, the examples of hemodynamic sensors described herein are applicable to both hemodynamic sensor 22 of IMD 14 (FIG. 1) are also applicable to hemodynamic sensor 52 of IMD 34.

In some examples, programmer 42 may be similar to external device 16 of monitoring system 10 (FIG. 1). In addition, a user may use programmer 42 to program a therapy progression, select electrodes used to deliver defibrillation pulses, select waveforms for the defibrillation pulse, or select or configure a fibrillation detection algorithm for IMD 34. The user may also use programmer 42 to program aspects of other therapies provided by IMD 34, such as cardioversion or pacing therapies. In some examples, the user may activate certain features of IMD 34 by entering a single command via programmer 42, such as depression of a single key or combination of keys of a keypad or a single point-and-select action with a pointing device.

IMD 34 and programmer 42 may communicate via wireless communication using any techniques known in the art. Examples of communication techniques may include, for example, low frequency or radiofrequency (RF) telemetry, but other techniques are also contemplated. In some examples, programmer 42 may include a programming head that may be placed proximate to the patient's body near the IMD 34 implant site in order to improve the quality or security of communication between IMD 34 and programmer 42.

FIG. 3 is a block diagram of an example IMD 14. In the example shown in FIG. 3, IMD 14 includes hemodynamic sensor 22, processor 60, memory 62, EGM sensing module 64, telemetry module 66, and power source 68. Memory 62 includes computer-readable instructions that, when executed by processor 60, cause IMD 14 and processor 60 to perform various functions attributed to IMD 14 and processor 60 herein. Memory 62 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

Processor 60 may include one or more microprocessors, controllers, digital signal processors (DSPs), application specific integrated circuits (ASICs), field-programmable gate arrays (FPGAs), or equivalent discrete or integrated logic circuitry, or combinations thereof. In some examples, processor 60 may include multiple components, such as any combination of one or more microprocessors, one or more controllers, one or more DSPs, one or more ASICs, or one or more FPGAs, as well as other discrete or integrated logic circuitry. The functions attributed to processor 60 herein may be embodied as software, firmware, hardware or any combination thereof. Processor 60 may control EGM sensing module 64 to sense EGM signals of heart 32 of patient 12 (FIG. 2) and may store EGM signals from EGM sensing module 64 in memory 62.

In some examples, hemodynamic sensor 22 may include two or more optical emitters for producing at least two different wavelengths of light, while in other examples, sensor 22 may include a single optical emitter that produces light at a single wavelength. In the example shown in FIG. 3, hemodynamic sensor 22 includes optical emitter 70, optical emitter 72, and detector 74, which may include one detector element or a plurality of detector elements (not shown). Optical emitters 70, 72 may be optical emitters of sensor 22. In particular, optical emitters 70, 72 may emit light, and, in some examples, may emit light having different wavelengths. In some examples, optical emitters 70, 72 may comprise a light emitting diode (LED), a laser diode, a vertical cavity surface emitting laser device, and the like.

Optical emitters 70, 72 are optically coupled to light transmission member 76, such that light emitted by optical emitters 70, 72 transmits through light transmission member 76 and exits at distal end 76A. Light transmission member 76, as well as the other light transmission members described herein, may comprise an optically conductive material, and may comprise a passive light transmitting member that does not require power to operate. For example, light transmission member 76 may comprise an optical fiber or an optical wave guide that guide light by total internal reflection. In examples in which light transmission member 76 comprises an optical fiber, the optical fiber may be configured to transmit light in a broad range of wavelengths, e.g., to transmit the light emitted by optical emitters 70, 72.

Light transmission member 76 may be mechanically coupled to housing 24 of IMD 14 using any suitable technique. In some examples, light transmission member 76 may be coupled to housing using interlocking components, an adhesive, welding (e.g., ultrasonic welding) or may be laminated into housing 24 (e.g., by being incorporated into a layer of housing 24), or light transmission member 76 may extend through housing 24 via a hermetic feedthrough and mechanically couple to an optical coupler 75, as described in further detail below.

In the example shown in FIG. 3, distal end 76A of light transmission member 76 defines a light emission portion. For example, light transmission member 76 may comprise an optical fiber and distal end 76A may include an opening (e.g., a cleaved end) through which light may exit the fiber. In other examples, light transmission member 76 may define a light emission portion in addition to or instead of distal end 76A. For example, light transmission member 76 may comprise multiple fibers defining multiple propagation paths for light, and one light emission portion may be positioned near a portion 76B of light transmission member 76 that is adjacent to housing 24 of IMD 14, or near a middle portion of light transmission member 76, which may be between proximal portion 76B and distal end 76A.

In some examples, optical emitters 70, 72 may emit light at different times, rather than substantially simultaneously. In other examples, optical emitters 70, 72 may emit light substantially simultaneously if detector 74 comprises detector elements configured to sense light at the particular wavelengths of light emitted by optical emitter 70 and optical emitter 72.

In the example shown in FIG. 3, optical emitters 70, 72 are coupled to light transmission member 76 via optical coupler 75. In some examples, optical coupler 75 may comprise a lens or an integrating sphere type assembly. In other examples, an optical fiber, waveguide or another optically transmissive member may couple optical emitters 70, 72 to light transmission member 76. For examples, two optical fibers may be fused together or otherwise coupled together to define a 2 x 1 optical coupler. Examples of suitable waveguides comprise waveguides formed of silicon or glass and comprises an optically transparent material such as lithium niobate. In other examples, optical emitters 70, 72 may be positioned next to the input aperture of light transmission member 76.

Optical coupler 75 may selectively couple optical emitters 70, 72 to light transmission member 76, depending upon which optical emitters 70, 72 is activated. In other examples, optical emitters 70, 72 may be coupled to separate light transmission members, rather than a common member 76 as shown in FIG. 3. Separate light transmission members may reduce the size and number of components inside of the IMD.

In some examples, optical emitter 70 may emit light in the red portion of the visible light spectrum, and optical emitter 72 may emit IR light in the IR portion of the light spectrum. Optical emitter 70 may emit light in the red portion of the visible light spectrum, such as, but not limited to, light having a wavelength in a range of about 550 nanometers (nm) to about 750 nm. Optical emitter 72 may emit IR light in the IR portion of the light spectrum, such as, but not limited to, light having a wavelength in a range of about 750 nm to about 2.5 micrometers or greater. In some examples, optical detector 74 may include at least one detector element that is sensitive to wavelengths of light in the red portion of the visible spectrum, and at least one detector element that is sensitive to wavelengths of light in the IR portion of the light spectrum. In other examples, detector 74 may include one or more detector elements that are configured to sense light in both the red portion and IR portion of the light spectrum.

Detector 74 may include, for example, a photodetector, such as a photodiode. In some examples, detector 74 may convert light incident on a detection surface of detector 74 into either a current or voltage, which may be outputted as an electrical signal. An intensity of the signal received by detector 74 may be indicative of hemodynamic function of patient 12, such as oxygen saturation of blood or the blood pressure of patient 12. In the example shown in FIG. 3, light transmission member 77 transmits light from tissue adjacent to distal end 77A of light transmission member 77 to the detection surface of detector 74. In this way, distal end 77A of light transmission member 77 provides a surrogate detection surface for detector that permits the detector 74 to, in effect, be located at a position that is at the surface of the housing of the IMD. In the example shown in FIG. 3, light transmission member 77 does not extend from housing 24. Instead, distal end 77A of light transmission member 77 is positioned at an outer surface of housing 24. In addition, distal end 77A of light transmission member 77 is not movable relative to housing 24 in the example shown in FIG. 3. In other examples, detector 74 may be directly coupled to an outer surface of housing 24, rather than optically coupled to the outer surface of housing 24 via light transmission member 77. In addition, as described in further detail below with respect to FIG 8, in some examples, detector 74 may be optically coupled to a light transmission member that extends from housing 24, which facilitates detection of light at a location displaced from housing 24.

Detector 74 is configured to sense light emitted by optical emitters 70, 72 that has passed through light transmission member 76, and transmitted through blood-perfused tissue of patient 12, which may be disposed in space 78 between distal end 76A of light transmission member 76 and the distal end 77A of light transmission member 77. The emitted light may transmit through, for example, a blood mass in an artery or other vasculature of patient 12. Optical emitters 70, 72 may emit light at light emission portion 76A of member 76, and the emitted light may scatter through tissue. Light that is transmitted through tissue within space 78 may be incident on distal end 77A of light transmission member 77, and light transmission member 77 may guide the light to detector 74.

Light transmission member 76 may extend from housing 24 any suitable distance. In the example shown in FIG. 3, light transmission member 76 extends distance D from housing 24, which may be about 7 millimeters (mm) to about 10 mm. However, other distances are contemplated. In the example shown in FIG. 3, light transmission member 76 is movable relative to housing 24, such that the location at which optical emitters 70, 72 emit light into tissue is adjustable. In this way, light transmission member 76 that extends from housing 24 and is optically coupled to optical emitters 70, 72 may help define a hemodynamic sensor that is adaptable to different implant sites within patient 12.

When a clinician implants IMD 14, the clinician may position light transmission member 76 such that vasculature of patient 12 is proximate detector 74 and distal end 76A of light transmission member 76, such as between detector 74 and distal end 76A of light transmission member 76 (e.g., within space 78). This may help increase the amount of light emitted by optical emitters 70, 72 that is transmitted through a blood mass of patient 12 prior to being detected by detector 74. Increasing the amount of light emitted by red optical emitters 70, 72 that is transmitted through a blood mass of patient 12 that is detected by detector 74 may help increase the signal-to-noise ratio of hemodynamic sensor 22.

Light transmission member 76 may be, but need not be, positioned such that distal end 76A of light transmission member 76 directly opposes detector 74, or, in the example shown in FIG. 3, directly opposes distal end 77A of light transmission member 77 that is coupled to detector 74. When optical emitters 70, 72 emit light, the light propagates through light transmission member 76 and into tissue, which scatters the light. Due to the scattering properties of the tissue, light may transmit from distal end 76A of light transmission member 76 through tissue and to detector 74 despite distal end 76A not being directly opposite the photodetection surface of detector 74 (e.g., directly opposite distal end 77A of light transmission member 77).

In some cases, an implant site for IMD 14 may not be proximate to vasculature of patient 12 or proximate to a satisfactory blood mass for determining relative changes in the blood oxygen saturation of patient 12. Hemodynamic sensor 22 including a light transmission member 76 that is readily conformable to different positions relative to detector 74 may help customize hemodynamic sensor 22 to different implant sites within patient 22, e.g., to position detector 74 and light transmission member 76 such that vasculature of patient 12 and/or a satisfactory blood mass for monitoring blood oxygen saturation levels of patient 12 is positioned between the light emission portion 76A of light transmission member 76 and detector 74. A clinician may manipulate the relative position between the light emission portion 76A of hemodynamic sensor 22 and detector 74 during implantation of IMD 14 within patient 12. In addition, with the aid of light transmission member 76, the clinician may select the effective distance between optical emitters 70, 72 and detector 74, which may dictate, for example, the volume of tissue through which light transmits before being sensed by detector 74.

In some cases, a suitable location proximate to blood-perfused tissue for hemodynamic sensor 22 may be an unsuitable implant site for IMD 14 due to the size and/or shape of housing 24 of IMD 14. In the example shown in FIG. 3, light transmission member 76 is smaller than housing 24, e.g., has a smaller cross-sectional size than housing 24. The cross-sectional sizes of light transmission member 76 and housing 24 may be, for example, the size at a cross-section taken at the largest portion of the light transmission member 76 and housing 24, respectively. For example, the cross-sectional size of light transmission member 76 may be taken along a direction substantially transverse to a longitudinal axis (as indicated by line 79 in FIG. 3) of light transmission member 76 when light transmission member 76 is substantially straight, and a cross-sectional size of housing 24 may be taken along a direction substantially transverse to a longitudinal axis (as indicated by line 25 in FIG. 1) of housing 24.

Due to the smaller size of light transmission member 76 and the movability of a distal portion 76A of light transmission member 76 relative to housing 24, at least a portion of hemodynamic sensor 22 may be positioned in a smaller space than housing 24 of IMD 14. This may increase the number of suitable implant sites for IMD 14, while still maintaining a suitable location of hemodynamic sensor 22 proximate to blood-perfused tissue.

The small size of light transmission member 76 may also help minimize the invasiveness of hemodynamic sensor 22. In some examples, light transmission member 76, as well as the other light transmission members described herein, may have a size that permits the light transmission member to be implanted in patient 12 without requiring sutures to close wound in the tissue caused by the introduction of light transmission member into the tissue. In some examples, light transmission member 76 has a round cross-section with a diameter of less than about 0.2 millimeters (mm) (about 8 mils). However, other sizes and cross-sectional shapes of light transmission member 76 are contemplated. In examples in which light transmission member 76 comprises an optical fiber, the cross-section of light transmission member 76 may include a reflective core, cladding, and a protective jacket.

Minimizing the invasiveness of hemodynamic sensor 22 may help reduce blood loss during implantation of IMD 14 within patient 12, as well as reduce the amount of scar tissue that may encapsulate light transmission member 76. Reducing the amount of scar tissue that encapsulates distal end 76A of light transmission member 76 may help increase the accuracy and precision of hemodynamic sensor 22 in detecting changes in the blood oxygen saturation level of patient 12. The absorption of light by scar tissue does not change with the blood oxygen saturation level of patient 12. Accordingly, scar tissue is generally a poor indicator of blood oxygen saturation level of patient 12.

In other examples of IMD 14, light transmission member 76 may be substantially rigid, such that the position of distal end 76A of light transmission member 76 is not movable relative to housing 24. A substantially rigid light transmission member 76 may be useful in fixing the relative position of distal end 76A of light transmission member 76 relative to housing 24 of IMD 14. This may help maintain a known and consistent distance between distal end 76A of light transmission member 76, from which light is emitted into tissue, and detector 74. The known and consistent distance between detector 74 and distal end 76A of light transmission member 76 may enable processor 60 to more accurately determine blood oxygen saturation levels of patient 12 by minimizing the probability of detecting spurious blood oxygen saturation level changes that are attributable to a change in the volume of tissue through which light is emitted prior to be detected by detector 74. Changes in the volume of tissue through which light is emitted may result in a change in the optical properties of the tissue, which may falsely indicate changes in blood oxygen saturation level of patient 12. In addition, the relative distance between detector 74 and the light emission portion of hemodynamic sensor 22 (e.g., distal end 76A of light transmission member 76) may affect the intensity of light received by detector 74, which may affect the accuracy and precision with which the electrical signal from detector 74 indicates the relative changes in the blood oxygen saturation level of patient 12.

In contrast to a substantially rigid light transmission member 76, a substantially flexible light transmission member 76 may be more giving, which may cause less trauma to adjacent tissue. As patient 12 moves, IMD 14 may also move within patient 12. If light transmission member 76 is substantially rigid, the light transmission member may maintain its position, despite adjacent tissue moving.

Examples of flexible light transmission member 76 include, for example, substantially flexible optical fibers or a waveguides. Examples of substantially rigid light transmission members 76 include substantially rigid waveguides, e.g., waveguides formed from silicon or glass.

Processor 60 may receive an electrical signal generated by detector 74. Processor 60 may then determine relative changes the blood oxygen saturation level of the blood proximate to hemodynamic sensor 22 or other hemodynamic characteristics of patient 12 based on the signal generated by detector 74. In examples in which detector array 74 includes a photodiode, an electrical signal outputted by detector 74 may be directly or inversely proportional to the amount of light (e.g., the intensity of light) incident on the photodiode or incident on distal end 77A of light transmission member 77, which guides light from the outer surface of housing 24 of IMD 14 to detector 74. As with light transmission member 76, light transmission member 76 may guide light via, e.g., internal reflectance.

Although not shown in FIG. 3, in some examples, hemodynamic sensor 22 may include one or more optical elements, such as one or more lenses that helps focus light emitted from optical emitters 70, 72. For example, optical emitters 70, 72 may emit light through the one or more lenses, and detector 74 may detect light received through one or more lenses.

Hemodynamic sensor 22 may be subcutaneously or submuscularly implanted within patient 12 such that blood perfused tissue of patient 12 is located in space 78 between distal end 76A of light transmission member 76 and distal end 77A of light transmission member 77. In the example of FIG. 3, distal end 76A of light transmission member 76 is positioned on a different side of the blood perfused tissue as detector 74, such that detector 74 detects light emitted from optical emitters 70, 72 and transmitted through a blood mass within the tissue. This type of hemodynamic sensor may be referred to as a transmissive hemodynamic sensor.

Distal end 76A of light transmission member 76 may be secured in a particular position within patient 12 using any suitable means. In some examples, light transmission member 76 or at least distal end 76A may be secured to adjacent tissue with the aid of surgical adhesives or binders, including in some cases optically transmissive adhesives. Examples of adhesives include, but are not limited to, 2-octyl cyanoacrylate, fibrin glue, or any other type of substance that cures upon contact with water or another fluid present in the surrounding tissue at the implant site. In addition, the solidifying substance may be activated or cured from body heat or an electrical current delivered to the substance. As another example of how a position of light transmission member 76 may be substantially fixed within patient 12, light transmission member 76 or at least distal end 76A may include an outer surface promotes tissue ingrowth. For example, at least a portion of light transmission member 76 may be textured or include a substance to promote tissue ingrowth.

Processor 60 controls hemodynamic sensor 22 to detect oxygen saturation levels of blood within tissue disposed between to light transmission member 76 and distal end 77A of light transmission member 77. Processor 60 may store electrical signals generated by detector 74 or perfusion values derived from the electrical signals generated by detector 74 in memory 62. Processor 60 may control the operation of optical emitters 70, 72. In some examples, processor 60 may control optical emitters 70, 72 to sequentially emit light, such that only one of the optical emitters 70, 72 emits light at a time.

Processor 60 may also control the operation of detector 74. In some examples, processor 60 may determine an intensity of each wavelength of light emitted by the respective optical emitter 70, 72 by controlling detector 74 to sequentially sense light having the wavelength that was most recently emitted. If detector 74 includes a plurality of detector elements, the detector elements may sense light in series or in parallel. When the detector elements sense light in parallel, processor 60 may receive an electrical signal from the detector elements substantially simultaneously, which may be received by processor 60 as one signal or a plurality of separate signals. In examples in which the detector elements of detector 74 provide separate electrical signals to processor 60, processor 60 may determine which, if any, detector element is detecting the greatest intensity of light. This may enable processor 60 to selectively activate detector elements of detector 74 in order to, for example conserve energy.

In other examples, in order to separate the signals indicative of the red light and IR light, processor 60 may demodulate the electrical signal received from detector 74. Light sensed by detector 74 may include information about the intensity of red light emitted by optical emitter 70 and reflected by blood, as well as the intensity of IR light emitted by optical emitter 72 and reflected by blood.

EGM sensing module 64 is electrically coupled to electrodes 18, 20. Electrodes 18, 20 may be coupled to a surface of outer housing 24 (FIG. 1) of IMD 14 or may be otherwise coupled to housing 24 of IMD 14, e.g., with the aid of one or more medical leads that extend from housing 24. In some examples in which electrodes 18, 20 are coupled to a surface of outer housing 24 of IMD 14, electrodes 18, 20 may be formed by housing 24 (e.g., by exposed portions of an electrically conductive housing) or may be attached to the outer surface of housing 24. Housing 24 may be any suitable housing that encloses some of the components of IMD 14. In some examples, housing 24 may be a hermetic housing that hermetically seals at least processor 60, memory 62, EGM sensing module 64, telemetry module 66, and power source 68.

EGM sensing module 64 receives signals from at least one of electrodes 18, 20 in order to monitor electrical activity of heart 32 of patient 12 (FIG. 2). In other examples, EGM sensing module 64 may be electrically coupled to more than two electrodes. In some examples, EGM sensing module 64 may include a channel that comprises an amplifier with a relatively wide-band. Signals from sensing electrodes 18, 20 may be coupled to the wide-band amplifier and provided to a multiplexer. Thereafter, the signals may be converted to multi-bit digital signals by an analog-to-digital converter for storage in memory 62 as an EGM. In some examples, the storage of such EGMs in memory 62 may be under the control of a direct memory access circuit.

In some examples, processor 60 may employ digital signal analysis techniques to characterize the digitized cardiac signals stored in memory 62 to detect and classify the patient's heart rhythm from the electrical signals. Processor 60 may detect and classify the heart rhythm of patient 12 by employing any of the numerous signal processing methodologies known in the art. In other examples, processor 60 may not analyze the stored EGM signals, and such processing may be done by another processor, such as a processor within external device 16 (FIG. 1), programmer 42 (FIG. 2) or another external computing device. In some examples, processor 60 may generate and store marker codes indicative of different cardiac episodes that EGM sensing module 64 detects, and store the marker codes in memory 62 and/or transmit the marker codes to external device 16 or another external computing device. An example pacemaker with marker-channel capability is described in U.S. Patent No. 4,374,382 to Markowitz, entitled, "MARKER CHANNEL TELEMETRY SYSTEM FOR A MEDICAL DEVICE," which issued on February 15, 1983.

Telemetry module 66 includes any suitable hardware, firmware, software or any combination thereof for communicating with another device, such as external device 16 (FIG. 1) or programmer 42 (FIG. 2). Under the control of processor 60, telemetry module 66 may receive downlink telemetry from and send uplink telemetry to external device 16 or programmer 42 with the aid of an antenna, which may be internal and/or external. Processor 60 may provide the data to be uplinked to external device 16 and the control signals for the telemetry circuit within telemetry module 66, e.g., via an address/data bus. In some examples, telemetry module 66 may provide received data to processor 60 via a multiplexer.

The various components of IMD 14 are coupled to power source 68, which may include a rechargeable or non-rechargeable battery. A non-rechargeable battery may be selected to last for several years, while a rechargeable battery may be inductively charged from an external device, e.g., on a daily or weekly basis.

The block diagram shown in FIG. 3 is merely one example of an IMD 14. In other examples, IMD 14 may include a fewer or greater number of components. For example, in examples in which IMD 14 is incorporated with a medical device that delivers therapy to patient 12, IMD 14 may also include a therapy delivery module, such as an electrical stimulation generator (e.g., a neurostimulator) or a fluid pump.

Hemodynamic sensor 22 and EGM sensing module 64 are shown to be separate from processor 60 in FIG. 3. In other examples, processor 60 may include the functionality attributed to hemodynamic sensor 22 and/or EGM sensing module 64 herein. For example, hemodynamic sensor 22 and EGM sensing module 64 shown in FIG. 3 may include software executed by processor 60. If hemodynamic sensor 22 or EGM sensing module 84 includes firmware or hardware, hemodynamic sensor 22 or EGM sensing module, respectively, may be a separate one of the one or more processors 60 or may be a part of a multifunction processor. As previously described, processor 60 may comprise one or more processors.

In some examples, some of the components of IMD 14 shown in the example of FIG. 3 may be relocated in another device. For example, hemodynamic sensor 22 may be enclosed in a separate housing from EGM sensing module 64 or other components of IMD 14. That is, although hemodynamic sensor 22 is shown in FIG. 3 to be incorporated within a housing of IMD 14 that also encloses other components, such as processor 60 and EGM sensing module 64, in other examples, hemodynamic sensor 22 may be enclosed in a separate housing as part of a separate hemodynamic sensor 22. A hemodynamic sensor 22 that is enclosed in a separate housing from the IMD 14 housing may be mechanically coupled to IMD 14 or may be mechanically decoupled from IMD 14. For example, in some examples, hemodynamic sensor 22 including optical emitters 70, 72, light transmission 76, and detector 74 may be implanted within patient 12 at a separate location from IMD 14. In such examples, hemodynamic sensor 22 may communicate with IMD 14 via a wired connection or via wireless communication techniques, such as RF telemetry.

In yet other examples, at least a part of hemodynamic sensor 22 may be external to patient 12. For example, hemodynamic sensor 22 may monitor the blood oxygen saturation level of tissue of patient 12 through epidermis layer of patient (e.g., through skin on a finger, earlobe or forehead of patient 12). Hemodynamic sensor 22 may transmit the electrical signals generated by detector 74 that are indicative of the sensed intensity of red light and IR light to another device, such as IMD 14, external device 16 or programmer 42. In some examples, data from at least one of hemodynamic sensor 22 or EGM sensing module 64 may be uploaded to a remote server, from which a clinician or another user may access the data to analyze the patient's condition. An example of a remote server is a server provided via the Medtronic CareLink® Network, available from Medtronic, Inc. of Minneapolis, Minnesota.

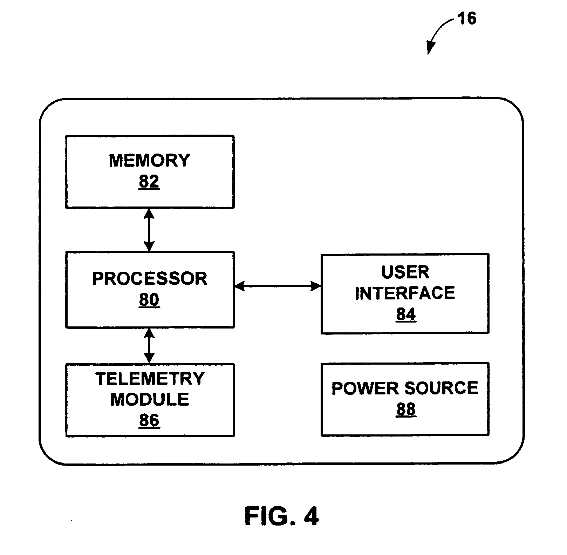

FIG. 4 is block diagram of an example external device 16. As shown in FIG. 4, external device 16 includes processor 80, memory 82, user interface 84, telemetry module 86, and power source 88. External device 16 may be a dedicated hardware device with dedicated software for interrogating IMD 14 to obtain information stored in memory 62 (FIG. 3), and, in some examples, for programming IMD 14. Alternatively, external device 16 may be an off-the-shelf computing device running an application that enables external device 16 to communicate with IMD 14.

A user may use external device 16 to modify the EGM and tissue perfusion sensing parameters of IMD 14. For example, the user may program the frequency at which EGM signals are sensed by EGM sensing module 64 (FIG. 3) or the tissue perfusion sensing time window for actively sensing changes in tissue perfusion with hemodynamic sensor 22. The clinician may interact with external device 16 via user interface 84, which may include display to present graphical user interface to a user, and a keypad or another mechanism for receiving input from a user.

Processor 80 can take the form of one or more microprocessors, DSPs, ASICs, FPGAs, programmable logic circuitry, or the like, and the functions attributed to processor 80 herein may be embodied as hardware, firmware, software or any combination thereof. Memory 82 may store instructions that cause processor 80 to provide the functionality ascribed to external device 16 herein, and information used by processor 80 to provide the functionality ascribed to external device 16 herein. Memory 82 may include any fixed or removable magnetic, optical, or electrical media, such as RAM, ROM, CD-ROM, hard or floppy magnetic disks, EEPROM, flash memory or the like. Memory 82 may also include a removable memory portion that may be used to provide memory updates or increases in memory capacities. A removable memory may also allow patient data to be easily transferred to another computing device, or to be removed before external device 16 is used to program therapy for another patient.

External device 16 may communicate wirelessly with IMD 14, e.g., using RF communication or proximal inductive interaction. This wireless communication is possible through the use of telemetry module 86, which may be coupled to an internal antenna or an external antenna. An external antenna that is coupled to external device 16 may correspond to the programming head that may be placed over the implant site of IMD 14, as described above with reference to FIG. 1. Telemetry module 86 may be similar to telemetry module 66 of IMD 14 (FIG. 3).

Telemetry module 86 may also be configured to communicate with another computing device via wireless communication techniques, or direct communication through a wired connection. Examples of local wireless communication techniques that may be employed to facilitate communication between external device 16 and another computing device include RF communication according to the 802.11 or Bluetooth specification sets, infrared communication, e.g., according to the IrDA standard, or other standard or proprietary telemetry protocols. In this manner, other external devices may be capable of communicating with external device 16 without needing to establish a secure wireless connection.

Power source 88 delivers operating power to the components of external device 16. Power source 88 may include a battery and a power generation circuit to produce the operating power. In some embodiments, the battery may be rechargeable to allow extended operation. Recharging may be accomplished by electrically coupling power source 88 to a cradle or plug that is connected to an alternating current (AC) outlet. In addition or alternatively, recharging may be accomplished through proximal inductive interaction between an external charger and an inductive charging coil within external device 16. In other embodiments, traditional batteries (e.g., nickel cadmium or lithium ion batteries) may be used. In addition, external device 16 may be directly coupled to an alternating current outlet to power external device 16. Power source 88 may include circuitry to monitor power remaining within a battery. In this manner, user interface 84 may provide a current battery level indicator or low battery level indicator when the battery needs to be replaced or recharged. In some cases, power source 88 may be capable of estimating the remaining time of operation using the current battery.

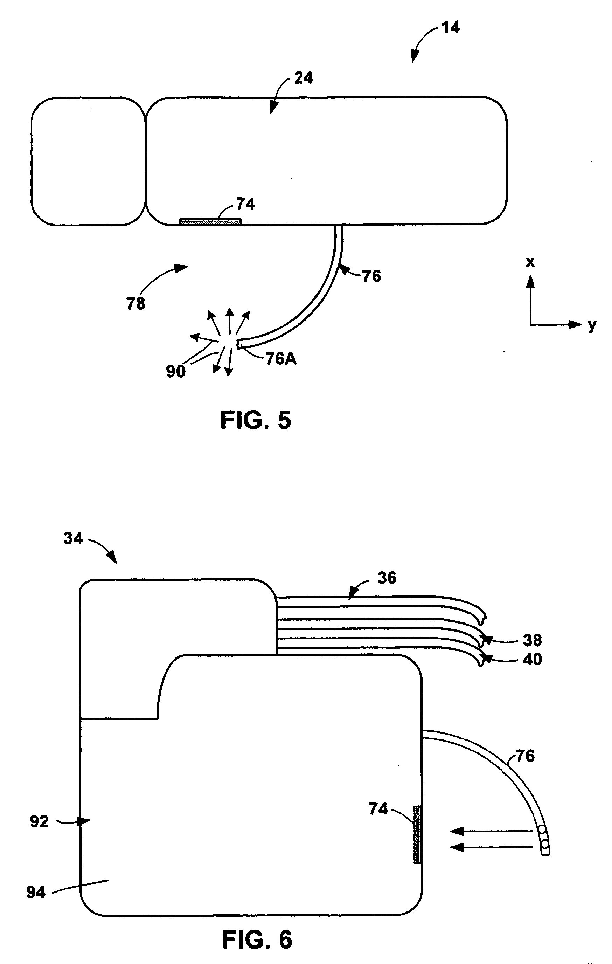

FIG. 5 is a conceptual illustration of an example IMD 14. In the example shown in FIG. 5, the photodetection surface of detector 74 is located at an outer surface of housing 24, rather than being optically coupled to outer surface of housing 24 with the aid of light transmission member 77, as shown in FIG. 3. For example, housing 24 may define a recess in which detector 74 is disposed, and one or more conductors may electrically couple detector 74 to processor 60 of IMD 14 (FIG. 3).

Light transmission member 76 extends from housing 24 of IMD 14 and is positioned such that light that is emitted from optical emitters 70, 72 (not shown in FIG. 5), which are optically coupled to light transmission member 76, may propagate through light transmission member 76 and into tissue adjacent to distal end 76A of light transmission member 76, as indicated by arrows 90. As shown in FIG. 5, at least some of the light guided away from housing 24 by light transmission member 76 may scatter through tissue within space 78 between distal end 76A of light transmission member 76 and an outer surface of housing 24. The light that transmits through tissue disposed in space 78 may be sensed by detector 74. Thus, detector 74 may generate an electrical signal that indicates the intensity of light emitted by optical emitters 70, 72 that has propagated through light transmission member 76 and through blood-perfused tissue of patient 12. The intensity of light sensed by detector 74 may indicate the amount of light that is transmitted through tissue within space 78 and not absorbed by blood within the tissue, which may indicate the relative blood oxygen saturation level of patient 12.

In some examples, light transmission member 76 may be substantially flexible (e.g., optical transmission member 76 may comprise an optical fiber), such that during implantation within patient 12, a clinician may manipulate light transmission member 76 and change the relative position between distal end 76A and detector 74. In this way, light transmission member 76 may permit hemodynamic sensor 22 to be customized to a particular anatomy of a patient 12 or to a particular implant site within patient 12. Light transmission member 76 may help configure hemodynamic sensor 22 such that blood-perfused tissue may be captured between a location at which light is emitted into tissue and a detector, while maintaining optical emitters 70, 72, and detector 74 within a biocompatible housing 24, which may be hermetically sealed. Thus, with the aid of light transmission member 76, hemodynamic sensor 22 may be a transmissive-type hemodynamic sensor 22 that is implantable within patient 12. In some examples, light transmission member 76 may be flexible in at least one of the x-axis, y-axis, and z-axis directions, where orthogonal x-y axes are shown in FIG. 5. In other examples, light transmission member 76 may be flexible in at least two of the x-axis, y-axis, and z-axis directions.

In some examples in which light transmission member 76 is substantially flexible and in some examples in which light transmission member 76 is substantially rigid, it may be desirable to fix a position of light transmission member 76 within patient 12 in order to help minimize migration of light transmission member 76. Migration of light transmission member 76 may change in relative position between the location at which light is emitted into patient 12 and detector 74, as well as the change in the relative position between hemodynamic sensor 22 and vasculature of patient 12. For at least these reasons, migration of light transmission member 76 may adversely affect performance of hemodynamic sensor 22 by changing the optical coupling between distal end 76A of light transmission member 76, from which light is emitted into tissue, and tissue of patient 12. For example, migration of light transmission member 76 may change the amount of tissue between distal end 76A and detector 74, which may change the optical characteristics of the tissue.

Light transmission member 76 may be secured in a particular position within patient 12 using any suitable means. In some examples, light transmission member 76 or at least distal end 76A may be secured to adjacent tissue with the aid of surgical adhesives or binders. Examples of adhesives include, but are not limited to, 2-octyl cyanoacrylate, fibrin glue, or any other type of substance that cures upon contact with water or another fluid present in the surround tissue at the implant site. In addition, the solidifying substance may be activated or cured from body heat or an electrical current delivered to the substance. As another example of how a position of light transmission member 76 may be relatively fixed within patient 12, light transmission member 76 or at least distal end 76A may include an outer surface promotes tissue ingrowth. For example, at least a portion of light transmission member 76 may be textured or include a substance to promote tissue ingrowth.

In other examples, light transmission member 76 may be relatively rigid, such that light transmission member 76 does not substantially move relative to housing 24 of IMD 14. A relatively rigid light transmission member 76 may also be, but need not be, fixed to adjacent tissue.

As previously described, an IMD that delivers therapy to patient 12 may also include hemodynamic sensor 22 including at least one of an optical emitter or detector coupled to a light transmission member that extends from a housing of the IMD. For example, as shown in FIG. 6, IMD 34 (FIG. 2), which includes a therapy delivery module for delivering electrical stimulation therapy to patient 12, includes light transmission member 76 extending from housing 92. Housing 92 may be biocompatible housing that encloses a therapy delivery module for delivering therapy to patient 12, and sensing circuitry for sensing one or more physiological parameters of patient 12, such as a blood oxygen saturation level of patient 12 and, in some cases, an EGM or ECG. Housing 92 may be hermetically-sealed. Also shown in FIG. 6 are leads 36, 38, 40, which are coupled to the therapy delivery module that is contained within housing 92 of IMD 34. Leads 36, 38,40 may each carry one or more electrodes for delivery of electrical stimulation to patient 12 or to sense one or more physiological parameters of patient 12. In addition, housing 92 may include an electrode for delivering electrical stimulation and/or sensing. Detector 74 may be directly coupled to outer surface 94 of housing 92 of IMD 14 or within a recess defined by housing 92. In other examples, detector 74 may be coupled to light transmission member 77 that collects light emitted by optical emitters 70, 72 and transmits the light to detector 74 that is disposed within housing 92.

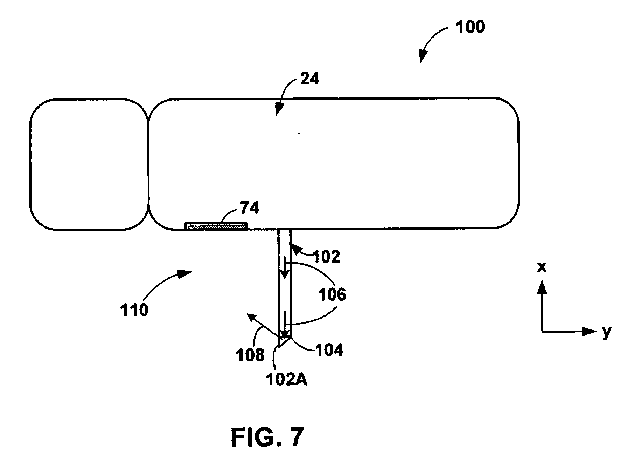

In some examples, a light transmission member may include a light deflection member that helps to direct light in a particular direction. FIG. 7 is a conceptual illustration of an example IMD 100, which includes light transmission member 102 extending from housing 24. IMD 100 is similar to IMD 14 of FIGS. 3 and 5, but includes a different light transmission member 102 optically coupled to optical emitters 70, 72. Just as with light transmission member 76 (FIG. 5), light transmission member 102 may be flexible in some examples or may be relatively rigid in other examples. In addition, in some examples, at least a portion of light transmission member 102 may be fixed to adjacent tissue.

As shown in FIG. 7, light transmission member 102 defines an opening at distal end 102A for emitting light into tissue proximate to distal end 102A. Light transmission member 102 includes light deflection member 104 proximate to distal end 102A. Light deflection member 104 may help direct light propagating through light transmission member in a particular direction away from distal end 102A of light transmission member 102. In the example shown in FIG 7, light transmission member 102 is adapted to guide light that is emitted by optical emitters 70, 72 toward light deflection member 104, as indicated by arrow 106. In addition, in the example shown in FIG. 7, light deflection member 104 is adapted to deflect light in a direction toward detector 74, as indicated by arrow 108. In some examples, light deflection member 104 may have a sufficiently higher index of refraction than tissue of patient 12, such that the beam of light propagating through light transmission member 102 may be totally internally reflected at the surface of light deflection member 104 and tissue of patient 12.

Light deflection member 104 may comprise, for example, one or more prisms or a surface within light transmission member 102 that is coated with a reflective material. In examples in which light deflection member 104 comprises a reflective surface, the light deflection member 104 may be defined by light transmission member 102 or a separate component that is attached to light transmission member 102. The index of refraction of light deflection member 104 may be selected to produce total internal reflection of the light beam at the interface of light deflection member 104 to tissue, helping direct light that exits distal end 102A of light transmission member 102 in a particular direction, such as toward detector 74, which is coupled to an outer surface of housing 24 in the example shown in FIG. 7.

In some examples, light deflection member 104 may be an optical component (e.g., a prism or a beam splitter comprising multiple prisms) that is also configured to split the light emitted by optical emitters 70, 72 into multiple light beams. This may help direct light in different directions within blood-perfused tissue of patient 12 that is disposed within space 110 between housing 24 and distal end 102A of light transmission member 102.

Light that is transmitted through a blood in tissue in space 110 between housing 24 and distal end 102A of light transmission member 102 may be sensed by detector 74. Processor 60 of IMD 100 (FIG. 3) may receive an electrical signal from detector 74 that indicates the intensity of light that is sensed by detector 74. Processor 60 may then determine various hemodynamic characteristics of patient 12 based on the electrical signal generated by detector 74, such as the relative oxygen saturation level of blood.

Light deflection member 104 enables optical emitters 70, 72 (FIG. 3) to transmit light through tissue between detector 74 and distal end 102A of light transmission member 102 without requiring distal end 102A of light transmission member 102 to directly oppose detector 74. Light deflection member 104 may be useful for increasing the number of acceptable positions of light transmission member 102 relative to housing 24 of IMD 100 when implanting IMD 100 within patient 12.

Although each of the above examples describe a light transmission member coupled to optical emitters 70, 72 and detector 74 that is on housing 24 of IMD 14 or is coupled to a light transmission member 77 (FIG. 3) that does not extend from housing 24 of IMD 14, in other examples, detector 74 may be coupled to a light transmission member that extends from housing 24 instead of or in addition to optical emitters 70, 72 that are optically coupled to a light transmission member that extends from housing 24.

FIG. 8 is a conceptual diagram of an example IMD 110, which is similar to IMD 14 of FIG. 3. Just as with IMD 14, IMD 110 includes processor 60, memory 62, EGM sensing module 64, telemetry module 66, and power source 68. Hemodynamic sensor 111 is similar to hemodynamic sensor 22 and includes optical emitters 70, 72, detector 74, and optical coupler 75. Optical emitters 70, 72 are coupled to light transmission member 112 via optical coupler 75, which may selectively coupled one of optical emitters 70, 72 to light transmission member 112. In addition, detector 74 is optically coupled to light transmission member 114. Light transmission members 112, 114 may each include an optical fiber or another optically conductive material.

Just as with light transmission member 76 (FIG. 3), light transmission member 112 guides light emitted by optical emitter 70 or optical emitter 72 away from housing 24 of IMD 110. Light transmission member 114 collects light emitted by re optical emitters 70, 72 by way of light transmission member 112 and guides the collected light to detector 74, which may be enclosed within housing 24. In the example shown in FIG. 8, light transmission members 112, 114 both extend from housing 24, such that both the optical emitters of IMD 110 and detector 74 may emit and detect light, respectively, at a tissue site that is further from housing 24 than would be permissible without light transmission members 112, 114. As described above, this may permit hemodynamic sensor 111 to detect blood oxygen saturation level changes in a blood mass (e.g., blood in an artery) of patient 12, despite the fact that housing 24 of IMD 110 may be too large or otherwise too cumbersome to be implanted near the blood mass.

Light transmission members 112, 114 are positioned such that blood-perfused tissue of patient 12 (e.g., a tissue site including a blood mass) is positioned in space 116 between distal ends 112A, 114A of light transmission members 112, 114, respectively. This arrangement between light transmission members 112, 114 may help define a transmissive-type hemodynamic sensor 111. That is, the arrangement between distal ends 112A, 114A of light transmission members 112, 114, respectively, may help detector 74 sense light emitted by optical emitters 70, 72 and transmitted through tissue in space 116.

In some examples, light transmission members 112, 114 may be movable relative to each other such that the effective distance between optical emitters 70, 72 and detector 74 may be customized to a particular implant site. In some cases, emitters 70, 72 and detector 74 may be positioned relatively close to each other within housing 24 due to, for example, the size constraints of implantable housing 24. The distance between the location at which light is emitted into tissue and the location at which the light is sensed by detector 74 may affect the ability of sensor 110 to sense changes in the patient's blood oxygen saturation level, tissue perfusion or other hemodynamic characteristics. For example, the greater the distance between the location at which light is emitted into tissue and the location at which light is sensed, the greater the volume of tissue is sampled by the sensor 110. Light transmission members 112, 114 permit the effective distance between emitters 70, 72 and detector 74 to be greater than the physical distance between the emitters 70, 72 and detector 74 within housing 24.

Light emitted by optical emitters 70, 72 may propagate through light transmission member 112, as indicated by arrows 120, and exit at distal end 112A of light transmission member 112. Light deflection member 118 at the distal end 112A of light transmission member 112 may help change the direction of the light emitted by optical emitter 70 or optical emitter 72, as indicated by arrow 122. In the example shown in FIG. 8, light deflection member 118 is configured to pivot the path of emitted light about 45 degrees to about 135 degrees, such as about 90 degrees, such that the emitted light may be directed at distal end 114A of light transmission member 114. In the example shown in FIG. 8, distal end 114A of light transmission member 114 is positioned to generally oppose distal end 112A of light transmission member 112, although distal ends 112A, 114A need not be lined up with each other (e.g., share spatial positions in two dimensions).

Light deflection member 118 may be similar to light deflection member 104 (FIG. 7) and may comprise any element that helps change a direction of light. For example, in some examples, light deflection member 118 may comprise one or more prisms, mirrors, or other surfaces within light transmission member 112 that comprise a reflective material. The reflective material may be a diffuse reflective material or a specular reflective material. The index of refraction of light deflection member 118 may be selected to help direct light in a particular direction, such as toward distal end 114A of light transmission member 114, as indicated by arrow 122 in FIG. 8.

Light incident on distal end 114A of light transmission member 114 may propagate through light transmission member 114, as indicated by arrows 124, and may be sensed by detector 74. In this way, detector 74 may sense light emitted by optical emitters 70, 72 at a location remote from housing 24, such as a tissue site proximate to an artery of patient 12. Light transmission member 114 includes light deflection member 126 that helps to direct light incident on distal end 114A of light transmission member 114 toward detector 74. Light deflection member 126 may be similar to light deflection member 104 described above with respect to FIG 7.

In the example shown in FIG 8, light deflection member 126 is configured to redirect the path of light that is approximately normal to a surface 126A of light deflection member 126, as indicated by arrow 128, about 45 degrees to about 135 degrees, such as about 90 degrees, such that the light emitted by optical emitters 70, 72 and transmitted through tissue may be guided toward detector 74 that is located at a proximal end 114B of light transmission member 114. That is, light deflection member 126 may help direct light toward housing 24 of IMD 110.

Light transmission members 112, 114 including respective light deflection members 118, 126 may help define a hemodynamic sensor 111 that may reflect and detect light closer to a blood mass of patient 12 without requiring housing 24 of IMD 110 to be implanted proximate to the blood mass. In addition, the relative small size of light transmission members 112, 114 (e.g., less than about 0.0254 mm) may help minimize the invasiveness of an implantable transmissive-type hemodynamic sensor 111.

Although the examples shown in FIGS. 5-8 illustrate a single light transmission member optically coupled to optical coupler 75, and a single light transmission member coupled to detector 74, this is by way of background only. In the claimed invention there are multiple light transmission members optically coupled to optical coupler 75 or directly to optical emitter 70 or optical emitter 72. The multiple light transmission members optically coupled to optical coupler 75 may, for example, define an array of light transmission members that are each configured to guide light emitted by one of optical emitter 70 or optical emitter 72 away from housing 24. The distal ends of the light transmission members may be arranged such that the light emitted at the distal ends is focused in particular direction. The distal ends of the light transmission members include light deflection members that further help define particular configuration of emitted light.

Similarly, in other examples, multiple light transmission members may be coupled to detector 74. In some examples, the light transmission members may each include a light deflection member that is configured to deflect light incident on the light deflection members of the different light transmission members. The light deflection members may be used to collect light and direct the light in a common direction. In some cases, at least some of the light deflection members may have different indices of refraction relative to each other, which may allow the light deflection members to help capture light that is incident on the light transmission members at different angles. This may allow detector 74 to capture light that is traversing through the patient's tissue at various angles, thereby increasing the quantity of light that detector 74 may sense.

FIG 9 is a schematic illustration of an example of hemodynamic sensor 130, according to the invention, which may be included in an IMD that also monitors cardiac signals of patient 12 (e.g., IMD 14 in FIG 1) or an IMD that delivers therapy to patient 12 (e.g., IMD 34 in FIG 2). Alternatively, hemodynamic sensor 130 may be a standalone (e.g., self-contained) device that is implanted in patient 12. Hemodynamic sensor 130 includes optical emitters 70, 72, detector 74, and optical coupler 75, which are described above with respect to FIG 3. Hemodynamic sensor 130 may also include a processor, memory, telemetry module, and power source (not shown in FIG 9), as described with respect to IMD 14 (FIG. 3).

Hemodynamic sensor 130 also includes light transmission members 132, 134, 136 that are optically coupled to optical emitters 70, 72 via optical coupler 75, and light transmission members 138, 140, 142 that are optically coupled to detector 74. Light transmission members 132, 134, 136, 138, 140, 142 may each comprise any suitable optically conductive material that may guide light from optical emitters 70, 72 away from housing 156 of hemodynamic sensor 130 or a housing of another IMD housing with which hemodynamic sensor 130 is incorporated. For example, light transmission members 132, 134, 136, 138, 140, 142 may comprise at least one of an optical fiber or a waveguide. In some examples, at least some of light transmission members 132, 134, 136, 138, 140, 142 may be substantially flexible, while in other examples, at least some of light transmission members 132, 134, 136, 138, 140, 142 may be substantially rigid.

Light deflection members 144, 146, 148 are positioned at distal ends 132A, 134A, 136A of light transmission members 132, 134, 136, respectively. In addition, light deflection members 150, 152, 154 are positioned at distal ends 138A, 140A, 142A of light transmission members 138, 140, 142, respectively. Light deflection members 144, 146, 148, 150, 152, 154 may each comprise any suitable reflective member that changes the path of light in a particular direction. Light deflection members 144, 146, 148, 150, 152, 154 may be each be similar to light deflection member 104 described above with respect to FIG. 7. For example, light deflection members 144, 146, 148, 150, 152, 154 may each comprise a prism, mirror or other surface coated with a reflective material. Light deflection members 144, 146, 148, 150, 152, 154 may each be formed, e.g., via micromachining of the respective light transmission member 132, 134, 136, 138, 140, 142 or may be attached to the respective light transmission member.

As described above, the index of refraction of light deflection members 144, 146, 148, 150, 152, 154 may be selected based on the angle of incidence of light on the light deflection member 144, 146, 148, 150, 152, 154 and the desired direction of light propagation from the respective light deflection member 144, 146, 148, 150, 152, 154.

In the example shown in FIG. 9, light transmission members 132, 134, 136 are substantially parallel, such that light emitted by optical emitters 70, 72 propagate through light transmission members 132, 134, 136 in substantially parallel paths. However, in other examples, light transmission members 132, 134, 136 may not be substantially parallel. Light transmission members 132, 134, 136 may be coupled to each other or may be freely movable relative to each other.

Distal ends 132A, 134A, 136A of light transmission members 132, 134, 136 define a geometrical array of openings through which light may exit light transmission members 132, 134, 136. The openings at distal ends 132A, 134A, 136A may define a two-dimensional (2D) array or a three-dimensional array (3D). In a 2D array, at least some of the openings may have different spatial positions in two dimensions. For example, at least some of the openings may lie in a common plane, but have different locations in two different dimensions. In a 3D array, at least some of the openings of the light transmission members 132, 134, 136 may have different spatial positions in at least three different dimensions. The openings at distal ends 132A, 134A, 136A of light transmission members 132, 134, 136 have different positions relative to housing 156 of hemodynamic sensor 130 in order to permit red optical emitters 70, 72 increase the diversity of locations at which light is emitted into tissue of patient 12. Increasing the number of locations and angles at which light is emitted into tissue may help increase the probability that detector 74 may sense light that has been transmitted through a blood mass. In addition, as described in further detail below, increasing the number of locations and angles at which light is collected by light transmission members 138, 140, 142 may help increase the amount of light emitted by optical emitters 70, 72 and transmitted through a blood mass that detector 74 detects.

Light deflection members 144, 146, 148 are each configured to reflect light in a different direction. As shown in FIG. 8, light deflection members 144, 146, 148 are each configured to deflect light emitted by optical emitters 70, 72 such that the light focuses towards openings at distal ends 138A, 140A, 142A of light transmission members 138, 140, 142, respectively. Although the light emitted by optical emitters 70, 72 may be scattered by tissue that is positioned between distal ends 132A, 134A, 136A of light transmission members 132, 134, 136 and distal ends 138A, 140A, and 142A of light transmission members 138, 140, 142, focusing the light toward ends 138A, 140A, and 142A of light transmission members 138, 140, 142 may help increase the amount of light that is transmitted to detector 74 via light transmission members 138, 140, 142. In some examples, at least two of the light deflection members 144, 146, 148 may have different indices of refraction.

In the example shown in FIG. 9, light transmission members 138, 140, 142 are substantially parallel, such that light received by light transmission members 138, 140, 142 propagate through light transmission members 138, 140, 142 in substantially parallel paths. However, in other examples, light transmission members 138, 140, 142 may not be substantially parallel. Light transmission members 138, 140, 142 may be coupled to each other or may be freely movable relative to each other. Distal ends 138A, 140A, and 142A of light transmission members 138, 140, 142 may define a geometrical array of openings through which light may enter light transmission members 138, 140, 142.

The openings at distal ends 138A, 140A, and 142A may define a 2D array or a 3D array. The openings at distal ends 138A, 140A, and 142A of light transmission members 138, 140, 142 have different spatial positions relative to housing 156 of hemodynamic sensor 130 in order increase the diversity of locations at which light is collected and transmitted to detector 74. Including a plurality of light transmission members 138, 140, 142 and positioning light transmission members 138, 140, 142 to define a 2D or 3D array of openings for receiving light may help to increase the probability that detector 74 of senses light that has been transmitted through a blood mass.

Distal ends 138A, 140A, and 142A of light transmission members 138, 140, 142 include light deflection members 150, 152, 154, respectively, that are each configured to deflect light incident on distal ends 138A, 140A, and 142A toward detector 74. The light emitted by optical emitters 70, 72 of hemodynamic sensor 130 may be scattered by the patient's tissue, and the emitted light that is transmitted through blood may not be focused in any particular direction. Thus, detector 74 coupled to one light transmission member may only receive a small percentage of the light that has been reflected by blood. Hemodynamic sensor 130 that includes detector 74 coupled to plurality of light transmission members 138, 140, 142 that collect light at various locations within tissue of patient 12 may increase the amount of light emitted by optical emitters 70, 72 and transmitted through a blood mass that detector 74 receives.

Due to the optical scattering properties of biological tissue, light emitted by optical emitters 70, 72 may be diffused upon entrance into the blood-perfused tissue proximate to distal ends 132A, 134A, 136A of light transmission members 132, 134, 136. The light emitted by optical emitters 70, 72 may be transmitted through blood and scattered at various angles. Thus, the light that is incident on distal ends 138A, 140A, and 142A of light transmission members 138, 140, 142, respectively, may have various angles of incidence. Diversifying the location and angles at which light transmission members 138, 140, 142 may receive light by diversifying the indices of refraction of light deflection members 150, 152, 154 may help increase the amount of light that detector 74 may sense, which may increase the sensitivity of hemodynamic sensor 22. Thus, in some examples, at least two of the light deflection members 150, 152, 154 may have different angles of incidence.

Increasing the angles at which light transmission members 138, 140, 142 collect light may help increase the quantity (or amount) of light that is detected by detector 74. In general, this may help increase the sensitivity of hemodynamic sensor 130 to changes in blood oxygen saturation levels of patient 12. In addition, collecting more reflected light by increasing the number of light transmission members 138, 140, 142 may help hemodynamic sensor 130 monitor the blood oxygen levels of a larger volume (e.g., a larger sample) of tissue. That is, because more light is captured by detector 74, the probability of detecting light that has transmitted through blood in different regions of tissue (e.g., in different blood vessels or different parts of the same blood vessel) may increase. This may help generate a better indication of the patient's blood oxygen saturation levels by increasing the quantity of light emitted by optical emitters 70, 72 that is received by detector 74 and/or increasing the volume of tissue that is sample by hemodynamic sensor 130.

Although three light transmission members 132, 134, 136 that extend from housing 156 of hemodynamic sensor 130 and guide light from optical emitters 70, 72 away from housing 156 are shown in FIG. 9, in other examples, any suitable number of light transmission members may be optically coupled to optical emitters 70, 72. In addition, although three light transmission members 138, 140, 142 that collect light emitted by red optical emitters 70, 72 and transmitted through blood-perfused tissue, and guide the light to detector 74 are shown in FIG. 9, in other examples, any suitable number of light transmission members may be optically coupled to detector 74. In addition, in some examples, the plurality of light transmission members coupled to optical emitters 70, 72 or detector 74 may not include light deflection members.

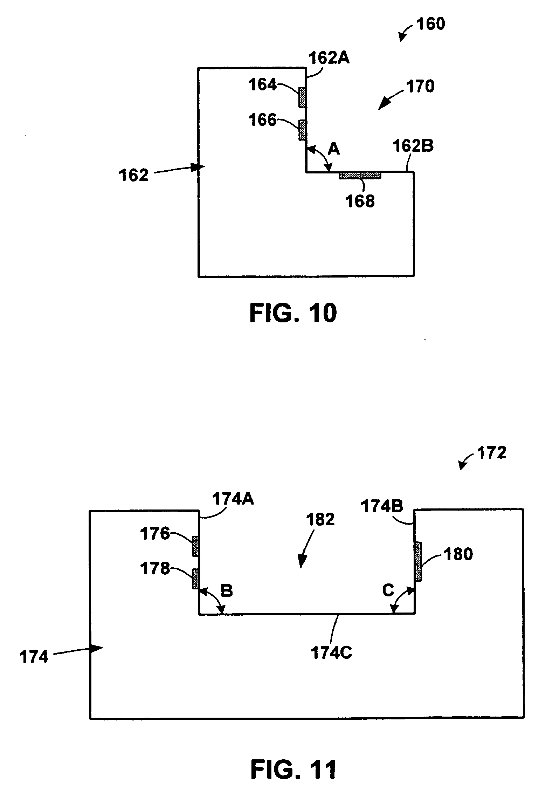

Although the hemodynamic sensors described with respect to FIGS. 3 and 5-9 each include a light transmission member that extends from a housing of an IMD in order to direct light away from the housing or to collect light and transmit the light toward the housing, in other examples, an implantable transmissive-type hemodynamic sensor may include other configurations. FIGS. 10-12 are conceptual illustrations of example implantable transmissive-type hemodynamic sensors.

FIG. 10 is a conceptual illustration of IMD 160, which includes housing 162 and an implantable hemodynamic sensor comprising red optical emitter 164, IR optical emitter 166, and detector 168. Red optical emitter 164, IR optical emitter166, and detector 168 may be substantially similar to optical emitters 70, 72, and detector 74, respectively, which are described above with respect to IMD 14. In other examples, optical emitters 164, 166 may emit light having any suitable wavelength.

Just as with IMD 14, IMD 160 may be an implantable monitor that does not provide therapy to patient 12 or may be configured to deliver stimulation to the heart of patient 12 or to deliver another type of therapy to patient 12 (e.g., delivery of a therapeutic agent). Housing 162 may substantially enclose various components of IMD 160, such as a processor, a memory, a telemetry module, a power source, and the like. The components of IMD 160 may be similar to the components of IMD 14, which are described above with respect to FIG. 3. In some examples, housing 162 may be a hermetic housing.

Housing 162 defines a first surface 162A and a second surface 162B that are oriented at an angle A relative to each other. In some examples, angle A may be about 10 degrees to about 180 degrees, such as about 30 degrees to about 135 degrees or about 90 degrees. Angles A may be selected such that blood-perfused tissue of patient may be positioned within space 170 defined between surfaces 162A, 162B. When implanting IMD 160 in patient 12, a clinician may orient housing 162 such that vasculature of patient 12 or another blood mass of patient suitable for monitoring a blood oxygen saturation level of patient 12 is positioned within space 170. Accordingly, angle A may be selected such that light emitted by red optical emitter 164 and IR optical emitter 166, which are positioned along first surface 162A, may transmit through blood-perfused tissue of patient located within space 170 prior to being sensed by detector 168, which is positioned along second surface 162B. Angle A may be selected to minimize the possibility that light emitted by red optical emitter 162 and IR optical emitter 162 transmits directly to detector 168 without first passing through tissue of patient 12. In this way, IMD 160 may include a transmissive-type hemodynamic sensor. Detector 168 may generate an electrical signal that changes as a function of the intensity of light transmitted through the tissue within space 170 and incident on photodetection surface of detector 168.

Red optical emitter 164 and IR optical emitter 166 may be positioned in a recess defined by housing 162 or may be directly coupled to an outer surface of housing 162. Similarly, detector 168 may be positioned in a recess defined by housing 162 or may be directly coupled to an outer surface of housing 162. Red optical emitter 164, IR optical emitter 166, and detector 168 may be directly or indirectly electrically coupled to a processor or other electrical components within housing 162 with the aid of hermetic feedthroughs. In other examples of IMD 160, any suitable number of optical emitters may be coupled to surface 162A and any suitable number of detectors may be coupled to surface 162B.

FIG. 11 is a conceptual illustration of an example IMD 172, which includes housing 174 and an implantable hemodynamic sensor comprising red optical emitter 176, IR optical emitter 178, and detector 180. Red optical emitter 176, IR optical emitter 178, and detector 180 may be substantially similar to optical emitters 70, and detector 74, respectively, which are described above with respect to IMD 14. In other examples, optical emitters 176, 178 may emit light having any suitable wavelength. Just as with IMD 14, IMD 172 may be an implantable monitor that does not provide therapy to patient 12 or may be configured to deliver therapy (e.g., stimulation and/or fluid delivery) to patient 12. Housing 174 may substantially enclose various components of IMD 172, such as a processor, a memory, a telemetry module, a power source, and the like. The components of IMD 172 may be similar to the components of IMD 14, which are described above with respect to FIG. 3. In some examples, housing 174 may be a hermetic housing.

Housing 174 defines first surface 174A and second surface 174B that substantially oppose each other. In the example shown in FIG. 11, first surface 174A of housing 174 is substantially parallel to second surface 174B. However, nonparallel arrangements between first surface 174A and second surface 174B are contemplated. Housing 172 also defines third surface 174C that extends between first surface 174A and second surface 174B. In some examples, angle B between first surface 174A and third surface 174C may be about 90 degrees to about 180 degrees, such as about 90 degrees, and angle C between second surface 174B and third surface 174C may be also be about 90 degrees to about 180 degrees, such as about 90 degrees. Varying the values of angles B and C may permit the optical hemodynamic sensor shown in FIG. 11 to vary from a reflective to a transmissive type sensor.

Angles B and C may be selected that such surfaces 174A-174C define space 182 for receiving blood-perfused tissue of patient 12. When implanting IMD 172 in patient 12, a clinician may orient housing 174 such that vasculature of patient 12 or another blood mass of patient suitable for monitoring a blood oxygen saturation level of patient 12 is disposed within space 182. In addition, angles B and C may be selected such that light emitted by red optical emitter 176 and IR optical emitter 178 may transmit through tissue disposed within space 182 prior to being detected by detector 180. In this way, housing 174 of IMD 160 may help define a transmissive-type hemodynamic sensor that is implantable within patient 12.

Space 182 defined by housing 174 may be useful for capturing a predefined volume of tissue and stabilizing the tissue relative to IMD 172. Capturing a predefined volume of tissue may be useful for controlling the volume of blood-perfused tissue that is monitored by the hemodynamic sensor of IMD 172. In examples in which the hemodynamic sensor of IMD 172 monitors the relative changes in blood oxygen saturation level, monitoring a consistent volume of blood-perfused tissue may help IMD 172 more accurately and precisely monitor changes in the blood oxygen saturation level. In some cases, changes in the tissue volume that is monitored by the hemodynamic sensor of IMD 172 may erroneously be characterized as a change in blood oxygen saturation level of patient 12. For example, if the volume of tissue that hemodynamic sensor of IMD 172 monitors increases, the amount of blood that is monitored by the hemodynamic sensor may increase, which may be incorrectly characterized as a change in blood oxygen saturation level of patient 12. Thus, by maintaining a relatively constant volume of tissue within space 182, the changes in the blood oxygen saturation level monitored by the hemodynamic sensor may be more accurate and precise.

Stabilizing the tissue captured within space 182 relative to IMD 172 may help minimize relative motion between optical emitters 176, 178, detector 180, and blood-perfused tissue of patient 12, which may help minimize motion artifacts that are introduced into the electrical signal generated by detector 180. Motion between optical emitters 176, 178, detector 180, and proximate blood-perfused tissue may change the optical coupling between optical emitters 176, 178 and detector 180 and the blood-perfused tissue, which may result in incorrect blood oxygen saturation readings. For example, motion may cause spurious blood oxygen saturation level changes to be detected by IMD 172. Thus, by implanting IMD 172 such that a volume of blood-perfused tissue is captured in space 182, walls 174A-174C of housing 174 may help limit movement between optical emitters 176, 178, detector 180, and proximate blood-perfused tissue.

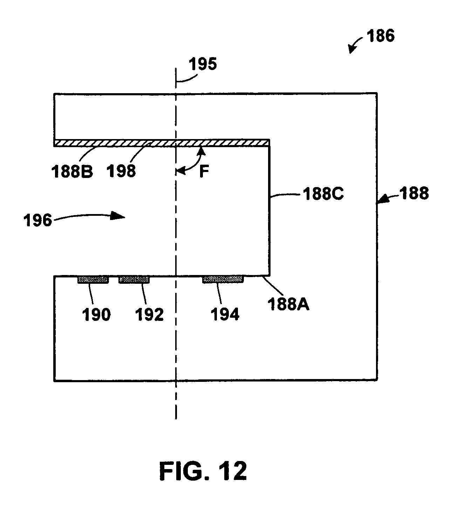

FIG. 12 is a conceptual illustration of an example IMD 186, which includes housing 188 and an implantable hemodynamic sensor comprising red optical emitter 190, IR optical emitter 192, and detector 194. Red optical emitter 190, IR optical emitter 192, and detector 194 may be substantially similar to optical emitters 70, 72, and detector 74, respectively, which are described above with respect to IMD 14. In other examples, optical emitters 190, 1292 may emit light having any suitable wavelength.

Just as with IMD 14, IMD 186 may be an implantable monitor that does not provide therapy to patient 12 or may be configured to deliver therapy (e.g., stimulation and/or fluid delivery) to patient 12. Housing 188 may substantially enclose various components of IMD 186, such as a processor, a memory, a telemetry module, a power source, and the like. The components of IMD 186 may be similar to the components of IMD 14, which are described above with respect to FIG. 3. In some examples, housing 188 may be a hermetic housing.

Housing 188 defines first surface 188A and second surface 188B that substantially oppose each other. In the example shown in FIG. 12, first surface 188A of housing 174 is substantially parallel to second surface 188B. However, nonparallel arrangements between first surface 188A and second surface 188B are contemplated. Housing 188 also defines third surface 188C that extends between first surface 188A and second surface 188B. Just as with IMD 172 of FIG. 11, surfaces 188A-188C of housing 188 define a space 196 that may be useful for capturing a predefined volume of tissue and stabilizing the tissue relative to IMD 186.

Red optical emitter 190, IR optical emitter 192, and detector 194 are positioned on first surface of housing 188A. For example, red optical emitter 190, IR optical emitter 192, and detector 194 may be disposed within a common or separate recesses defined by surface 188A of housing 188 or may be directly coupled (e.g., via an adhesive or welding) to surface 188A. A reflective surface 198 is placed along at least a part of second surface 188B of housing 188 that opposes first surface 188A. Reflective surface 198 may comprise polished titanium or sputtered gold over a titanium substrate. In other examples, reflective surface 198 may comprise a diffuse reflective material or a specular reflective material.

Light emitted by red optical emitter 190 and IR optical emitter 192 may transmit through blood-perfused tissue within space 196, and reflective surface 198 may reflect the light back to detector 194. Detector 194 may then generate an electrical signal that indicates an intensity of light that is emitted by optical emitters 190, 192 and transmitted through tissue within space 196.

Optical axis 195 defines a path of light from red optical emitter 190 and IR optical emitter 192 to reflective surface 198, and from reflective surface 198 to detector 194. In some examples, surface 188B of housing 188 and red optical emitter 190, IR optical emitter 192, and detector 194 are arranged such that optical axis 195 is substantially normal to a major surface of reflective surface 198. Such an orientation of optical axis 195 relative to reflective surface 198 may permit the most the most energy (e.g., the most light emitted by optical emitters 190, 192) to be specularly reflected to the detector 194. In other examples, an angle F between optical axis 195 and a major surface of reflective surface 198 may be about 45 degrees to about 135 degrees.

In the example shown in FIG 12, light emitted by optical emitters 190, 192 passes through tissue in space 196 twice, first from optical emitters 190, 192 toward reflective surface 198, and subsequently from reflective surface 198 towards surface 188A of housing 188. The reflective surface 198 may be rigidly attached to the housing 188 or may be a separate piece that is implanted in close proximity to housing 188. The light that transmits through the blood-perfused tissue within space 196 twice may help increase the sensitivity of the hemodynamic sensor of IMD 186 to changes in the blood oxygen saturation level of patient 12. In particular, the increase in the optical path would result in a greater interaction of the light with the blood in the tissue. This increased interaction would improve the ability of the pulse oximeter to resolve (e.g., sense) smaller changes in blood oxygen.

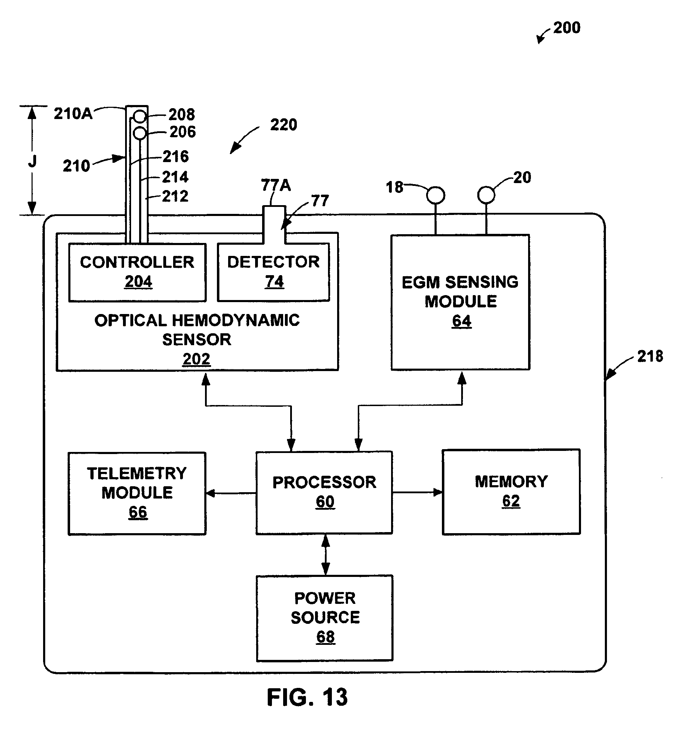

FIG. 13 is a conceptual illustration of another example of an IMD 200 that includes an optical hemodynamic sensor for sensing a hemodynamic characteristic of patient 12, such as an arterial blood oxygen saturation level, arterial blood flow, tissue perfusion, and the like. IMD 200 includes processor 60, memory 62, EGM sensing module 64, telemetry module 66, power source 68, and optical hemodynamic sensor 202. Processor 60, memory 62, EGM sensing module 64, telemetry module 66, and power source 68 are described above with respect to IMD 14 (FIG 3).

Optical hemodynamic sensor 202 comprises detector 74, which is optically coupled to light transmissive member 77, controller 204, optical emitters 206, 208, and flexible circuit 210. Optical emitters 206, 208 are carried by an extension member that extends from housing 218 of IMD 200. In particular, optical emitters 206, 208 are mechanically and electrically coupled to flexible circuit 210, which electrically couples controller 204 to optical emitters 206, 208. Housing 218 may comprise an implantable, biocompatible, and hermetic housing in some examples. In the example shown in FIG. 13, flexible circuit 210 extends from housing 218 of IMD 200 and optical emitters 206, 208 are carried at a distal end 210A of flexible circuit 210. In some examples, flexible circuit 210 may comprise an elongated shape, e.g., having a greater length than width in cross-section. Thus, in some examples, flexible circuit 210 may define an elongated member. Other types of elongated members or extension members that extend from housing 218 and electrically couple emitters 206, 208 to one or more components within housing 218 are contemplated.

Flexible circuit 210 may comprises flexible backing 212 and conductive traces 214, 216 that electrically couple optical emitters 206, 208, respectively, to controller 204. In some examples, backing 212 may comprise a flexible, electrically insulating polymer, such as polyimide. Traces 214, 216 may comprise any suitable electrically conductive material.

In some examples, optical emitters 206, 208 may each comprise a LED, a laser diode, a vertical cavity surface emitting laser device, or the like. Emitters 206, 208 of IMD 200 are external to housing 218 of IMD 200. In the example shown in FIG. 13, emitters 206, 208 are located at a distal end of flexible circuit 210. Emitters 206, 208 may be mechanically coupled to flexible circuit 210 using any suitable biocompatible technique, such as, but not limited to, an adhesive, welding, crimping, conductive and nonconductive epoxies, solders, and the like. In some examples, emitters 206, 208 may be positioned on flexible circuit 210 such that emitters 206, 208 substantially face housing 218 of IMD 230 and/or distal end 77A of light transmission member 77.

Flexible circuit 210 has a substantially smaller cross-sectional size than housing 218 of IMD 200, which may permit emitters 206, 208 to be implanted proximate to vasculature or another blood mass of patient 12, even if housing 218 of IMD 200 is too large or otherwise unsuitable for implantation proximate to the blood mass. The cross-sectional sizes of flexible circuit 210 and housing 218 may be, for example, the size measured at the largest cross-section of flexible circuit 210 and housing 218, respectively.

The relatively small size of flexible circuit 210 may help minimize the invasiveness of hemodynamic sensor 202. As with light transmission member 76 of IMD 14 (FIG. 3), flexible circuit 210 may have a size that permits circuit 210 and emitters 206, 208 to be implanted in patient 12 without requiring sutures to close wound in the tissue caused by the introduction of light transmission member into the tissue. Minimizing the invasiveness of hemodynamic sensor 202 may help reduce blood loss during implantation of IMD 200 within patient 12, as well as reduce the amount of scar tissue that may encapsulate emitters 206, 208. Reducing the amount of scar tissue that encapsulates emitters 206, 208 may help decrease the power that is required to operate sensor 200, as well as increase the accuracy and precision of hemodynamic sensor 200 in detecting changes in the blood oxygen saturation level of patient 12.

Controller 204 may comprise one or more microprocessors, DSPs, ASICs, FPGAs, or any other equivalent integrated or discrete logic circuitry. Controller 204 may control the operation of optical emitters 206, 208, such as, for example, providing power to one or both of the emitters 206, 208 via conductive traces 214, 216 in order to cause the respective emitter 206, 208 to emit light. Processor 60 may control the operation of optical emitters 206, 208 via controller 204. For example, processor 60 may control the frequency with which emitters 206, 208 emit light or the order in which emitters 206, 208 emit light if the emitters emit light at different times. In some examples, processor 60 may directly control the operation of emitters 206, 208, and, therefore, may include the functionality attributed to controller 204 herein.

Detector 74 is configured to sense light emitted by optical emitters 206, 208 that has transmitted through blood-perfused tissue of patient 12, which may be disposed in space 220 between distal end 210A of flexible circuit 210 and distal end 77A of light transmission member 77. The emitted light may transmit through, for example, a blood mass in an artery or other vasculature of patient 12. Optical emitters 206, 208 may emit light at the interface between tissue of patient 12 and emitters 206, 208. Light that is transmitted through tissue within space 220 may be incident on distal end 77A of light transmission member 77, and light transmission member 77 may guide the light to detector 74. As previously described, detector 74 may generate an electrical signal that changes as a function of the intensity of light that is received by detector 74. Processor 60 may receive the electrical signal from detector 74 and determine one or more hemodynamic characteristics of patient 12 based on the electrical signal.

In some cases, an interface between a light transmission member (e.g., light transmission member 76 shown in FIG. 3) and optical emitters may result in some loss of light (or energy), which may be a significant loss in some cases. Accordingly, by directly emitting light into tissue of patient 12, rather than emitting light via a light transmission member (e.g., light transmission member 76 of FIG. 3), IMD 200 may minimize the amount of light (or energy) that is lost between emitters 206, 208 and tissue. Minimizing the loss of light between emitters 206, 208 and tissue of patient 12 may help decrease the power that optical hemodynamic sensor 202 consumes in order to sense one or more hemodynamic characteristics of patient 12. Furthermore, increasing the light that is transmitted into tissue of patient 12 may help improve the signal-to-noise ratio of optical hemodynamic sensor 202.

Flexible circuit 210 may extend from housing 218 any suitable distance. In the example shown in FIG. 13, flexible circuit 210 extends distance J from housing 218, which may be about 7 mm to about 10 mm. However, other distances are contemplated. In the example shown in FIG. 13, flexible circuit 210 is substantially flexible and movable relative to housing 218, such that the location of optical emitters 206, 208 relative to detector 74 is adjustable. In this way, emitters 206, 208 that are external to housing 24 and coupled to a flexible circuit 210 may help define a hemodynamic sensor that is adaptable to different implant sites within patient 12. As with optically transmissive member 76 (FIG. 3), flexible circuit 210 may enable a clinician to implant IMD 200 such that vasculature of patient 12 or other blood-perfused tissue of patient 12 is disposed within space 220 between emitters 206, 208 and detector 74.

In some examples, flexible circuit 210 may be secured to adjacent tissue with the aid of tissue ingrowth, surgical adhesives or binders, including in some cases optically transmissive adhesives. Examples of adhesives include, but are not limited to, 2-octyl cyanoacrylate, fibrin glue, or any other type of substance that cures upon contact with water or another fluid present in the surrounding tissue at the implant site.

In other examples, flexible circuit 210 may be secured to adjacent tissue with the aid of compressive forces from adjacent tissue. For example, when flexible circuit 210 and optical emitters 206, 208 are implanted within patient 12, a clinician may guide the circuit 210 and emitters 206, 208 to an implant site with the aid of an insertion tool, such as a needle introducer defining an inner lumen sized to receive flexible circuit 210 and emitters 206, 208. For example, the clinician may first introduce the insertion tool into tissue of patient 12 and subsequently guide flexible circuit 210 and emitters 206, 208 through the inner lumen of the introducer to the implant site. Alternatively, the clinician may insert the insertion tool with the flexible circuit 210 and emitters 206, 208 already introduced in the inner lumen. Upon withdrawal of the insertion tool, flexible circuit 210 and emitters 206, 208 may remain implanted within patient 12 in an insertion path within the tissue defined by the insertion tool. The pressure of tissue adjacent the insertion path defined by the insertion tool may hold flexible circuit 210 and emitters 206, 208 substantially in place. A flexible circuit 210 that may move with patient 12 movement may help minimize the invasiveness of optical hemodynamic sensor by decreasing friction or other undesirable interaction between flexible circuit 210 and adjacent tissue.

In other examples of IMD 200, emitters 206, 208 may be electrically coupled to controller 204 with the aid of a substantially rigid electrically conductive member.

In some examples, a detector of an implantable hemodynamic sensor may also be external to a housing of an IMD that includes various IMD components, such as a processor and memory. The detector may be carried by an extension member that extends from the housing of the IMD. For example, the extension member may comprise a substantially flexible or a substantially rigid member that also electrically couples the detector to components with the IMD housing.

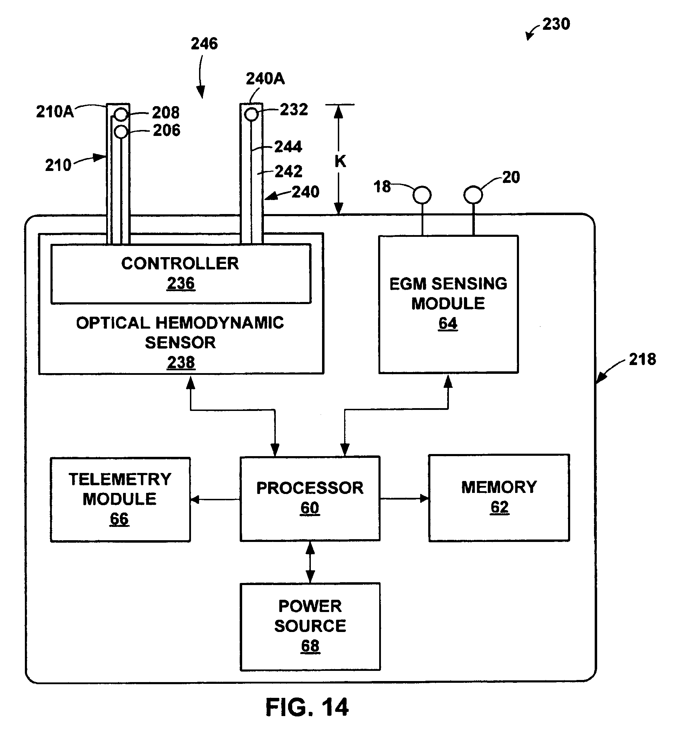

FIG. 14 is a conceptual illustration of IMD 230 that includes detector 232 that is external to housing 218 of IMD 230 and electrically coupled to controller 236 of optical hemodynamic sensor 238 with flexible circuit 240. As with IMD 200 of FIG. 13, emitters 206, 208 are external to housing 218 and electrically coupled to controller 236 via flexible circuit 210. In the example shown in FIG. 14, detector 232 is located at a distal end 240A of flexible circuit 240. Flexible circuit 240 includes flexible, electrically insulating backing 242 and an electrically conductive trace 244 that is electrically coupled to detector 232 and controller 236. Detector 232 may be any suitable optical detector, such as a photodiode. Detector 232 may be mechanically coupled to flexible circuit 240 using any suitable biocompatible technique, such as, but not limited to, an adhesive, welding, crimping, conductive and nonconductive epoxies, solders, and the like. In the example shown in FIG. 14, emitters 206, 208 may be positioned on flexible circuit 210 and detector 232 may be positioned on flexible circuit 210 such that emitters 206, 208 and detector 232 generally face each other. A clinician may also manipulate flexible circuits 210, 240 during implantation within patient 12 such that emitters 206, 208 and detector 232 generally face each other (e.g., generally oppose each other in some examples), although flexible circuits 210, 240 need not extend the same distance from housing 218.

Flexible circuit 240 has a substantially smaller cross-sectional size than housing 218 of IMD 230, which may permit detector 232 to be implanted proximate to vasculature or another blood mass of patient 12, even if housing 218 of IMD 200 is too large 3or otherwise unsuitable for implantation proximate to the blood mass. The cross-sectional sizes of flexible circuit 240 may be, for example, the size measured at the largest cross-section of flexible circuit 240.

Flexible circuit 240 may extend from housing 218 any suitable distance. In the example shown in FIG. 14, flexible circuit 240 extends distance K from housing 218, which may be about 7 mm to about 10 mm. However, other distances are contemplated. In the example shown in FIG 14, flexible circuits 210, 240 are substantially flexible and movable relative to housing 218, such that the volume of tissue between detector 74 and emitters 206, 208 is adjustable. Although flexible circuits 210, 240 are substantially parallel in FIG. 14, in other examples, flexible circuits 210, 240 may be nonparallel relative to each other.

Detector 232 that extends from housing 218 of IMD 230 with the aid of flexible circuit 240 may help increase the flexibility of optical hemodynamic sensor 238 to accommodate different implant sites within patient 12, as well as different anatomical structures of different patients. During implantation of IMD 230 within patient 12, a clinician may adjust the relative position of flexible circuits 210, 240 in order to modify the volume of tissue between emitters 206, 208 and detector 232, as well as the location of emitters 206, 208 and detector 232 relative to vasculature of patient 12. Thus, the clinician may control the volume of tissue through which light transmits prior to being detected by detector 232. This may help customize optical hemodynamic sensor 238 to patient 12.

By directly sensing light that is emitted by emitters 206, 208 and transmitted through tissue of patient 12, rather than sensing light that has first transmitted through a light transmission member (e.g., light transmission member 114 of FIG. 8), IMD 230 may minimize the amount of light (or energy) that is lost as a result of the interface between the light transmission member and detector 232.

In some examples, flexible circuit 240 may be secured to adjacent tissue with the aid of tissue ingrowth, surgical adhesives or binders, including in some cases optically transmissive adhesives, as previously described with respect to flexible circuit 210. In other examples, flexible circuit 240 may be secured to adjacent tissue with the aid of compressive forces from adjacent tissue. For example, as with flexible circuit 210, flexible circuit 240 and detector 232 may be introduced into patient 12 with the aid of an insertion tool that defines an insertion path through tissue. Upon withdrawal of the insertion tool, flexible circuit 240 and detector 232 may remain implanted within the insertion path and the pressure of tissue adjacent the insertion path defined by the insertion tool may hold flexible circuit 240 and detector 232 substantially in place.

Controller 236 may comprise one or more microprocessors, DSPs, ASICs, FPGAs, or any other equivalent integrated or discrete logic circuitry. Controller 236 may control the operation of optical emitters 206, 208, such as, for example, providing power to one or both of the emitters 206, 208 in order to cause the respective emitter 206, 208 to emit light. In addition, controller 236 may control the operation of detector 232. Processor 60 may control the operation of detector 232 via controller 236. For example, processor 60 may control the frequency with which detector 232 senses light emitted by emitters 206, 208. In some examples, processor 60 may directly control the operation of emitters 206, 208 and detector 232, and, therefore, may include the functionality attributed to controller 236 herein. Processor 60 may also receive electrical signals generated by detector 232 via flexible circuit 240 directly or indirectly, e.g., via controller 236

Detector 232 is configured to sense light emitted by optical emitters 206, 208 that has been transmitted through blood-perfused tissue of patient 12, which may be disposed in space 246 between distal end 210A of flexible circuit 210 and distal end 240A of flexible circuit 240. The emitted light may transmit through, for example, a blood mass in an artery or other vasculature of patient 12. Optical emitters 206, 208 may emit light at the interface between tissue of patient 12 and emitters 206, 208. Light that is transmitted through tissue within space 246 may be incident detector 232, which may generate an electrical signal that changes as a function of the intensity of light that is received by detector 232. Processor 60 may receive the electrical signal from detector 232 and determine one or more hemodynamic characteristics of patient 12 based on the electrical signal.

Although optical hemodynamic sensors comprising two optical emitters are described herein, in other examples, an optical hemodynamic sensor may comprise a single optical emitter or more than two optical emitters. The optical emitters may emit light having the same or different wavelengths.

The techniques described in this disclosure, including those attributed to IMD 14, external device 16, or various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as physician or patient programmers, stimulators, image processing devices or other devices. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

When implemented in software, the functionality ascribed to the systems, devices and techniques described in this disclosure may be embodied as a computer program, which may comprise instructions on a computer-readable medium such as RAM, ROM, NVRAM, EEPROM, FLASH memory, magnetic data storage media, optical data storage media, or the like. The instructions may be executed to support one or more aspects of the functionality described in this disclosure.

In other examples, the techniques described as being performed by processor 60 of IMD 14 may be performed in whole or in part by processor 80 of external device 16 or another device. For example, processor 80 of external device 16 may receive an electrical signal generated by detector 74 of IMD 14 and determine a physiological parameter value of patient 12 based on the electrical signal.

## Claims

1. An implantable medical system comprising:
an implantable housing (156);
means for emitting light (70, 72), wherein the means for emitting light is coupled to the housing;
means for sensing light (74), wherein the means for sensing light is coupled to the housing and is configured to generate a signal indicative of an intensity of light emitted by the means for emitting light and transmitted to the means for sensing light; and
a plurality of light deflection members (144, 146, 148); **characterized by**
a plurality of light transmission members (132, 134, 136) extending different distances from the housing;
wherein at least one of the means for emitting light or the means for sensing light are optically coupled to the plurality of light transmission members, and wherein each of the light transmission members comprises an optical opening for at least one of receiving light and transmitting light to the housing or emitting light transmitted from the housing, and the plurality of light transmission members are arranged to define an array of optical openings having different spatial positions relative to the implantable housing; and
wherein the plurality of light deflection members (144, 146, 148) are optically coupled to a respective one of the light transmission members.

2. The implantable medical system of claim 1, wherein at least one of the light transmission members comprises at least one of an optical fiber or an optical waveguide.

3. The implantable medical system of any of claims 1 or 2, wherein the means for emitting light comprises at least one of a light emitting diode, a laser diode or a vertical cavity surface emitting laser.

4. The implantable medical system of any of claims 1-3, wherein at least one of the plurality of light transmission members is movable relative to the housing.

5. The implantable medical system of any of claims 1-3, wherein at least one of the plurality of light transmission members is substantially rigid.

6. The implantable medical system of any of claims 1-5, wherein the light transmission members each have a smaller cross-sectional size than the housing.

7. The implantable medical system of any of claims 1-6, wherein the plurality of light transmission members comprises a first set of light transmission members, the system further comprising a second set of light transmission members, wherein the means for emitting light is optically coupled to the first set of light transmission members and the means for sensing light is optically coupled to the second set of light transmission members.

8. The implantable medical system of any of claims 1-6, wherein at least two of the light transmission members of the plurality of light transmission members are substantially parallel to one another.

9. The implantable medical system of claim 1, wherein the light deflection member comprises at least one of a prism, mirror or a surface of the light transmission member comprising a reflective material.

10. The implantable medical system of any of claims 1-6, wherein at least two of the light deflection members have different indices of refraction.

11. The implantable medical system of any of claims 1-10, further comprising a processor (60) that receives the signal generated by the means for sensing light and determines a hemodynamic characteristic of a patient based on the signal, wherein the processor is disposed within the implantable housing.

12. The implantable medical system of claim 11, wherein the hemodynamic characteristic comprises a blood oxygen saturation level, blood flow, a hematocrit level, or tissue perfusion.

13. The implantable medical system of any of claims 1-12, further comprising a surgical adhesive or binder that secures at least a portion of the light transmission member to tissue of a patient.

14. The implantable medical system of any of claims 1-6 or 7-13, wherein the plurality of light transmission members are arranged to define a two-dimensional or a three-dimensional array of optical openings having different spatial positions relative to the implantable housing.

## Patentansprüche

1. Implantierbares medizinisches System, mit:
einem implantierbaren Gehäuse (156);
Mitteln zum Aussenden von Licht (70, 72), wobei die Mittel zum Aussenden von Licht mit dem Gehäuse gekoppelt sind;
Mitteln zum Erfassen von Licht (74), wobei die Mittel zum Erfassen von Licht mit dem Gehäuse gekoppelt sind und konfiguriert sind, um ein Signal zu erzeugen, das eine Stärke von durch die Mittel zum Aussenden von Licht ausgesendetem und zu den Mitteln zum Erfassen von Licht gesendetem Licht angibt; und
mehreren Lichtablenkelementen (144, 146, 148); **gekennzeichnet durch**
mehrere Lichtdurchlasselemente (132, 134, 136), die sich über unterschiedliche Strecken von dem Gehäuse erstrecken;
wobei wenigstens eines der Mittel zum Aussenden von Licht oder der Mittel zum Erfassen von Licht mit den mehreren Lichtdurchlasselementen optisch gekoppelt ist und wobei jedes der Lichtdurchlasselemente eine optische Öffnung aufweist, um Licht zu empfangen und Licht zu dem Gehäuse durchzulassen und/oder um Licht, das von dem Gehäuse gesendet wird, auszusenden, und die mehreren Lichtdurchlasselemente angeordnet sind, um eine Anordnung optischer Öffnungen zu definieren, die verschiedene räumliche Positionen relativ zu dem implantierbaren Gehäuse haben; und
wobei die mehreren Lichtablenkelemente (144, 146, 148) mit einem Entsprechenden der Lichtdurchlasselemente optisch gekoppelt sind.

2. Implantierbares medizinisches System nach Anspruch 1, wobei wenigstens eines der Lichtdurchlasselemente eine Lichtleitfaser und/oder einen Lichtwellenleiter enthält.

3. Implantierbares medizinisches System nach einem der Ansprüche 1 oder 2, wobei die Mittel zum Aussenden von Licht eine Leuchtdiode und/oder eine Laserdiode und/oder einen oberflächenemittierenden Laser mit vertikalem Hohlraum enthalten.

4. Implantierbares medizinisches System nach einem der Ansprüche 1-3, wobei wenigstens eines der mehreren Lichtdurchlasselemente relativ zu dem Gehäuse beweglich ist.

5. Implantierbares medizinisches System nach einem der Ansprüche 1-3, wobei wenigstens eines der mehreren Lichtdurchlasselemente im Wesentlichen starr ist.

6. Implantierbares medizinisches System nach einem der Ansprüche 1-5, wobei die Lichtdurchlasselemente jeweils eine kleinere Querschnittsgröße als das Gehäuse haben.

7. Implantierbares medizinisches System nach einem der Ansprüche 1-6, wobei die mehreren Lichtdurchlasselemente eine erste Gruppe von Lichtdurchlasselementen enthalten, wobei das System ferner eine zweite Gruppe von Lichtdurchlasselementen enthält, wobei die Mittel zum Aussenden von Licht mit der ersten Gruppe von Lichtdurchlasselementen optisch gekoppelt sind und die Mittel zum Erfassen von Licht mit der zweiten Gruppe von Lichtdurchlasselementen optisch gekoppelt sind.

8. Implantierbares medizinisches System nach einem der Ansprüche 1-6, wobei wenigstens zwei der Lichtdurchlasselemente der mehreren Lichtdurchlasselemente im Wesentlichen zueinander parallel sind.

9. Implantierbares medizinisches System nach Anspruch 1, wobei das Lichtablenkelement ein Prisma und/oder einen Spiegel und/oder eine Oberfläche des Lichtdurchlasselements, die ein reflektierendes Material aufweist, enthält.

10. Implantierbares medizinisches System nach einem der Ansprüche 1-6, wobei wenigstens zwei der Lichtablenkelemente verschiedene Brechungsindizes haben.

11. Implantierbares medizinisches System nach einem der Ansprüche 1-10, das ferner einen Prozessor (60) enthält, der das von den Mitteln zum Erfassen von Licht erzeugte Signal empfängt und eine hämodynamische Eigenschaft eines Patienten anhand des Signals bestimmt, wobei der Prozessor in dem implantierbaren Gehäuse angeordnet ist.

12. Implantierbares medizinisches System nach Anspruch 11, wobei die hämodynamische Eigenschaft einen Blutsauerstoffsättigungsgrad, einen Blutfluss, einen Hämatokritgrad oder eine Gewebeperfusion enthält.

13. Implantierbares medizinisches System nach einem der Ansprüche 1-12, das ferner einen chirurgischen Klebstoff oder ein chirurgisches Bindemittel enthält, das wenigstens einen Abschnitt des Lichtdurchlasselements an Gewebe eines Patienten befestigt.

14. Implantierbares medizinisches System nach einem der Ansprüche 1-6 oder 7-13, wobei die mehreren Lichtdurchlasselemente so angeordnet sind, dass sie eine zweidimensionale oder eine dreidimensionale Anordnung optischer Öffnungen definieren, die unterschiedliche räumliche Positionen relativ zu dem implantierbaren Gehäuse haben.

## Revendications

1. Système médical implantable comportant :
un boîtier implantable (156) ;
des moyens d'émission de lumière (70, 72), les moyens d'émission de lumière étant couplés au boîtier ;
des moyens de détection de lumière (74), les moyens de détection de lumière étant couplés au boîtier et étant configurés pour générer un signal indicatif d'une intensité de lumière émise par les moyens d'émission de lumière et
transmis aux moyens de détection de lumière ; et
une pluralité d'éléments de déviation de lumière (144, 146, 148) ; **caractérisé par**
une pluralité d'éléments de transmission de lumière (132, 134, 136) prolongeant différentes distances à partir du boîtier ;
dans lequel au moins un parmi les moyens d'émission de lumière ou les moyens de détection de lumière sont optiquement couplés à la pluralité d'éléments de transmission de lumière, et dans lequel chacun des éléments de transmission de lumière comporte une ouverture optique pour au moins une action parmi la réception de lumière et la transmission de lumière au boîtier ou l'émission de lumière transmise à partir du boîtier, et la pluralité d'éléments de transmission de lumière est agencée pour définir un réseau d'ouvertures optiques ayant différentes positions spatiales par rapport au boîtier implantable ; et
dans lequel la pluralité d'éléments de déviation de lumière (144, 146, 148) est optiquement couplée à un élément respectif parmi les éléments de transmission de lumière.

2. Système médical implantable selon la revendication 1, dans lequel au moins un des éléments de transmission de lumière comporte au moins un élément parmi une fibre optique ou un guide d'ondes optique.

3. Système médical implantable selon l'une quelconque des revendications 1 ou 2, dans lequel les moyens d'émission de lumière comportent au moins un élément parmi une diode électroluminescente, une diode laser ou un laser à cavité verticale émettant par la surface.

4. Système médical implantable selon l'une quelconque des revendications 1 à 3, dans lequel au moins un élément parmi la pluralité d'éléments de transmission de lumière est mobile par rapport au boîtier.

5. Système médical implantable selon l'une quelconque des revendications 1 à 3, dans lequel au moins un élément parmi la pluralité d'éléments de transmission de lumière est sensiblement rigide.

6. Système médical implantable selon l'une quelconque des revendications 1 à 5, dans lequel les éléments de transmission de lumière ont chacun une taille de section transversale plus petite que le boîtier.

7. Système médical implantable selon l'une quelconque des revendications 1 à 6, dans lequel la pluralité d'éléments de transmission de lumière comporte un premier ensemble d'éléments de transmission de lumière, le système comportant en outre un second ensemble d'éléments de transmission de lumière, dans lequel les moyens d'émission de lumière sont optiquement couplés au premier ensemble d'éléments de transmission de lumière et les moyens de détection de lumière sont optiquement couplés au second ensemble d'éléments de transmission de lumière.

8. Système médical implantable selon l'une quelconque des revendications 1 à 6, dans lequel au moins deux des éléments de transmission de lumière de la pluralité d'éléments de transmission de lumière sont sensiblement parallèles l'un à l'autre.

9. Système médical implantable selon la revendication 1, dans lequel l'élément de déviation de lumière comporte au moins un élément parmi un prisme, un miroir ou une surface de l'élément de transmission de lumière comportant un matériau réfléchissant.

10. Système médical implantable selon l'une quelconque des revendications 1 à 6, dans lequel au moins deux des éléments de déviation de lumière ont des indices de réfraction différents.

11. Système médical implantable selon l'une quelconque des revendications 1 à 10, comportant en outre un processeur (60) qui reçoit le signal généré par les moyens de détection de lumière et détermine une caractéristique hémodynamique d'un patient sur la base du signal, dans lequel le processeur est disposé à l'intérieur du boîtier implantable.

12. Système médical implantable selon la revendication 11, dans lequel la caractéristique hémodynamique comporte un niveau de saturation d'oxygène dans le sang, un flux sanguin, un niveau d'hématocrite ou une perfusion de tissu.

13. Système médical implantable selon l'une quelconque des revendications 1 à 12, comportant en outre un adhésif ou un liant chirurgical qui fixe au moins une partie de l'élément de transmission de lumière sur un tissu d'un patient.

14. Système médical implantable selon l'une quelconque des revendications 1 à 6 ou 7 à 13, dans lequel la pluralité d'éléments de transmission de lumière est agencée pour définir un réseau bidimensionnel ou tridimensionnel d'ouvertures optiques ayant différentes positions spatiales par rapport au boîtier implantable.
